# EUROPEAN PATENT SPECIFICATION

(11) **EP 3 171 933 B1**
(45) Date of publication and mention of the grant of the patent: **14.08.2024**
(21) Application number: 15825209.8
(22) Date of filing: 23.07.2015
(51) Int. Cl.: A61N 1/30

(54) **PRECISION CHEMICAL ABLATION AND TREATMENT OF TISSUES**
CHEMISCHE PRÄZISIONSABLATION UND BEHANDLUNG VON GEWEBEN
ABLATION CHIMIQUE DE PRÉCISION ET TRAITEMENT DE TISSUS

(30) Priority: 23.07.2014 US 201462028013 P
(43) Date of publication of application: 31.05.2017
(73) Proprietor: Toth, Landy, Doylestown, PA 18901 (US); Schwartz, Robert, Inver Grove Heights, MN 55076 (US)
(72) Inventor: Toth, Landy, Doylestown, PA 18901 (US); Schwartz, Robert, Inver Grove Heights, MN 55076 (US)
(74) Representative: Page White Farrer
(86) International application number: PCT/US2015/041665
(87) International publication number: WO 2016/014750

(56) References cited:
- WO-A1-2006/007473
- US-A1- 2012 310 140
- US-A1- 2013 096 552

## Description

### CROSS-REFERENCE TO RELATED APPLICATION

The present application is a national stage of International Application PCT/US2015/041665 which claims the benefit of and priority to U.S. Provisional Application Ser. No. 62/028,013, filed on July 23, 2014, and entitled "Precision Chemical Ablation and Treatment of Tissues," by Landy Toth et al.

### BACKGROUND

### Technical Field

The present disclosure relates to the field of interventional modification of neurological or cardiac function of tissues. The present disclosure relates to compositions, systems, devices, and methods for performing neuromodulation, denervation, and/or ablation of tissues.

### Background

There are several disease states wherein ablation, neuromodulation, or functional change in a tissue is desired. Such disease states include pain management, arrhythmia treatments, neuroendocrine disorders, autoimmune disorders, lower urinary tract symptoms (LUTS), central nervous system disorders, and cancer.

Injections of therapeutic ablative agents are used to perform chemical ablations of tissues in order to treat such disease states. It is challenging to control the migration and affected zone with such agents, as they can migrate deep into surrounding tissues after injection therein and can lead to a range of complications.

There is a need to perform ablation, neuromodulation, or functional change of tissues with reduced complications.

US 2013 0096552 discloses a hydrodissection material that provides a flowable biocompatible material that increases in viscosity in situ to reduce material migration during an ablation procedure. US 2012 0310140 relates to the delivery of agents to target spinal and neuronal anatomies for the treatment of various disorders.

### SUMMARY

The invention provides a composition according to claim 1. A composition, comprising an ablative agent comprising ethyl alcohol for performing a treatment within a body of a subject, wherein the ablative agent represents more than 85% of the composition by mass; an excipient for limiting migration of at least one of the composition and the ablative agent within the body after delivery to the site, wherein the excipient comprises hydroxypropyl cellulose having an average molecular weight of approximately 1,000,000 or greater; and one or more surfactants; wherein the composition exhibits a change from a first set of properties in a first local environment comprising a syringe with a needle to a second set of properties in a second local environment comprising the tissue site; and wherein the first set of properties comprises a first viscosity and the second set of properties comprises a second viscosity, the second viscosity being higher than the first viscosity; and wherein the composition forms a low viscosity fluid at a temperature between 45 and 80° Celsius, and has a low shear rate viscosity of greater than 1000 mPa.s (cPs). The invention also provides a delivery system according to claim 8. A delivery system, comprising a delivery tool including a lumen, the lumen forming a fluid coupling between a distal end and a proximal end of the delivery tool, wherein the delivery tool including a lumen comprises a syringe with a needle; a reservoir for retaining a composition according to claims 1-7 prior to delivery of the composition to a treatment site within a volume of tissue, the reservoir coupled with the proximal end of the delivery tool; an injector coupled to the reservoir, the injector configured to deliver a bolus of the composition into the delivery tool upon activation thereof; and a delivery tip coupled to the lumen, the delivery tip deploy-ably coupled to the delivery tool, shaped and dimensioned so as to penetrate into or bias against the volume of tissue upon deployment from the delivery tool, the delivery tip comprising one or more ports coupled to the lumen, the ports arranged upon the delivery tip so as to access the treatment site. The invention further provides a
delivery system according to any of the claims 8-12 for use in a method for treating a region in a volume of tissues, and at least one of monitoring the effect of the composition on an electrophysiological state of a region in the volume of tissue and monitoring the migration of the composition in the region after delivery to the site, according to claim 13. The invention is defined by the claims. Any subject-matter falling outside the scope of the claims is provided for information purposes only.The references to methods of treatment in the summary and detailed description of the invention are to be interpreted as references to the delivery system according to claim 13 for use in a method for treating a region in a volume of tissues.

One illustrative, non-limiting objective of this disclosure is to provide a microsurgical tool for monitoring, evaluating the function of, mapping, and/or modulating electrophysiological activity in the vicinity of a lumen within a body. Another illustrative, non-limiting objective is to provide systems and methods for evaluating the extent of a neuromodulation procedure such as a neuromodulating surgery and/or stimulation. Yet another illustrative, non-limiting objective is to provide systems and methods for modifying lymphatic structures and the function or integrity thereof in a body.

According to a first aspect, there is provided an ablative composition for treatment of a site within a body of a subject including an ablative agent in accordance with the present disclosure for performing the treatment, and an excipient in accordance with the present disclosure for limiting migration of the composition and/or the ablative agent within the body after delivery to the site.

In aspects, the composition may include one or more components each in accordance with the present disclosure to facilitate the treatment, the delivery, the storage, the retention, and/or the stability of the composition.

The ablative agent includes ethyl alcohol,

The ablative agent represents more than 85% of the composition by mass. In aspects, a solvent may be added to the composition to adjust the low shear viscosity thereof.

The excipient comprises hydroxypropyl cellulose having an average molecular weight of approximately 1,000,000 or greater.

In aspects, the composition may be formulated so as to form a viscous thixotropic gel with a thixotropic index of greater than 1.25, greater than 1.5, greater than 2, or greater than 4, at 37°C (degrees Celsius) and/or a Bingham plastic with a yield strength of greater than 5 Pa (Pascals), greater than 20 Pa, or greater than 100 Pa, at 37°C. The composition forms a low viscosity fluid at a temperature between 45 and 80°C and has a low shear rate viscosity of greater than 1000 mPa.s (cPs). 4,000 mPa.s 2,000 mPa.s 500 mPa.s

The excipient comprises hydroxypropyl cellulose (HPC).

In aspects, the composition may be formulated so as to form a gel-like skin when submerged into an aqueous medium and is substantially soluble in a solution of the active agent.

In aspects, the ablative agent may act as a vehicle for the composition, the viscosity of the composition substantially increasing as the active agent migrates into a volume of tissues surrounding the site, after delivery to the site.

In aspects, the composition may be formulated so as to limit migration of the active agent from an injection site to a distance of less than approximately 3mm (millimeters), less than approximately 2mm, less than approximately 1mm, or the like from a margin of a bolus formed by the composition after delivery to the site within a timeframe comparable with the delivery of the composition to the site.

In aspects, the ablative composition may include a contrast agent selected from a fluorescent agent, a CT (computed tomography) contrast agent, an iodine based contrast agent, an MRI (magnetic resonance imaging) contrast agent, or a combination thereof.

In aspects, the ablative agent may include a chemotherapeutic agent, a cytotoxic agent, an antibody drug conjugate, an anti-neural growth factor, a mitotic inhibitor, a poison, a neurotoxin, a combination thereof, or the like.

There is provided a delivery system comprising a delivery tool including a lumen, the lumen forming a fluid coupling between a distal end and a proximal end of the delivery tool, wherein the delivery tool including a lumen comprises a syringe with a needle; a reservoir for retaining a composition according to claims 1-7 prior to delivery of the composition to a treatment site within a volume of tissue, the reservoir coupled with the proximal end of the delivery tool; an injector coupled to the reservoir, the injector configured to deliver a bolus of the composition into the delivery tool upon activation thereof; and a delivery tip coupled to the lumen, the delivery tip deploy-ably coupled to the delivery tool, shaped and dimensioned so as to penetrate into or bias against the volume of tissue upon deployment from the delivery tool, the delivery tip comprising one or more ports coupled to the lumen, the ports arranged upon the delivery tip so as to access the treatment site.

In aspects, the delivery system may include a thermal regulating unit coupled to the lumen and/or the reservoir, the thermal regulating unit configured to maintain the composition at a predetermined temperature prior to and/or during delivery. The thermal regulating unit may include a heating band, braid, laser machined hypotube, or the like coupled with the lumen, the heating band configured to maintain the composition at a temperature during delivery through the lumen.

In aspects, the ports may be arranged along the delivery tip with a spatially changing density and/or diameter such that the bolus may be shaped when delivered from the delivery tip.

In aspects, the delivery tip may include or may be a needle, the needle shaped so as to penetrate into the volume of tissue upon deployment, the ports arranged along the length of the needle. The ports may be arranged such that the bolus is formed substantially in the shape of a cylinder, a sphere, an ellipsoid, a torus, a tear drop, a cone, or the like when delivered to the site.

In aspects, the delivery system may include a balloon coupled with the delivery tip, the balloon coupled to a fluid source so as to be expand-ably deployed during a procedure so as to interface the delivery tip with the wall of a vessel or the volume of tissue. The balloon may include one or more energy delivery elements, and/or sensing elements to interface with the wall of the lumen and/or the volume of tissue.

In aspects, the delivery tool and/or the delivery tip may include one or more sensing elements, or electrodes each in accordance with the present disclosure to interface with the volume of tissue. In aspects, the system may be configured to direct energy through the energy delivery elements based upon the information collected by the sensing elements or electrodes. The sensing elements may be configured to monitor and/or determine the signals relating to regions of abnormal electrophysiological activity, determine the direction of nerve traffic along nerves in the volume of tissue, determine the sympathetic neural activity in the volume of tissue, determine the type of nerves situated near the sensing element, determine the effectiveness of the energy and/or composition delivery, determine the response of nerve traffic to a stress test performed on the body or the organ, determine the positioning of the sensing elements in the body, determine the transition of the sensing elements between anatomical features in the body (e.g., between a muscle and an adventitia, through a membrane, into a wall of an artery, etc.), a combination thereof, or the like.

In aspects, the volume of tissues may be coupled to one or more regions of a vessel wall, an artery, a vein, an arteriole, an adventitia of a vessel wall, an organ, a muscle mass, a ganglion, a diseased tissue, a tumor, combinations thereof, or the like.

In aspects, the delivery tip may have a characteristic diameter of less than 1mm, less than 0.75mm, less than 0.5mm, or less than 0.3mm so as to access the volume of tissue within the body.

In aspects, the system may include a tissue suction element, a deployable cup-like element, or the like in accordance with the present disclosure, coupled to the delivery tip, the suction element configured to retain the site against the delivery tip upon activation before, during, and/or after the delivery. In aspects, the suction element may be arranged so as to draw the site onto the delivery tip upon activation.

In aspects, the delivery tip may be arranged within the suction element so as to deliver the bolus into the drawn in site of the tissue.

According to aspects, not covered by the scope of the claims, there is provided use of a composition in accordance with the present disclosure and/or a system in accordance with the present disclosure to reduce, and/or prevent communication of pain signals originating within a tumor microenvironment or associated organ from traveling along a nerve in the volume of tissue.

According to aspects, not covered by the scope of the claims, there is provided use of a composition in accordance with the present disclosure and/or a delivery system in accordance with the present disclosure to treat a cardiac disease, a cardiac arrhythmia, to isolate a tissue site in a cardiac muscle, to treat a diseased tissue site in an organ, or a combination thereof.

According to aspects, not covered by the scope of the claims, there is provided use of a composition in accordance with the present disclosure and/or a delivery system in accordance with the present disclosure to form an embolism in a region of an organ, a kidney, a portion of a kidney served by an accessory vessel, or a combination thereof.

A delivery system according to any of the claims 8-12 for use in a method for treating a region in a volume of tissues, and at least one of monitoring the effect of the composition on an electrophysiological state of a region in the volume of tissue and monitoring the migration of the composition in the region after delivery to the site.

In aspects, the method may include forming a pattern of the composition in the region. The pattern may be formed in the shape of a ring around the perimeter of the region, so as to isolate the region from the surrounding volume of tissue, formed through deposition of a plurality of boluses at points over a three dimensional path within the volume of tissue.

In aspects, the region may include a tumor and the pattern may be formed over the margin of the tumor.

According to aspects, not covered by the scope of the claims, there is provided a method to ablate and/or assess a region of an organ coupled to an arterial tree including identifying a branch of the arterial tree that substantially exclusively provides blood flow to the region, and delivering a bolus of a composition in accordance with the present disclosure into the branch.

In aspects, not covered by the scope of the claims, the step of identifying may be facilitated by performing one or more contrast angiograms in one or more branches of the arterial tree, correlating an approach with a 3D (three dimensional) tomographic image, a CT image, an MRI image, etc.

In aspects, not covered by the scope of the claims, the method may include monitoring the effect of the composition on the electrophysiological state of the branch (e.g., so as to determine the state of nerve kill, nerve block, the completion of the ablation procedure, the electrophysiological response to a stress test, etc.).

In aspects, not covered by the scope of the claims, the method may include monitoring migration of the composition into the organ and/or a vascular tree coupled thereto.

In aspects, not covered by the scope of the claims, the organ may be a kidney, and the arterial tree may be coupled to an accessory artery.

In aspects, not covered by the scope of the claims, the method may include performing a stress test on the region of the organ, the stress test including injecting a drug, or a stressing agent such as a vasodilator, a vasoconstrictor, a neuroblocker, a neurostimulant, a diuretic, insulin, glucose, beta-adrenergic receptor antagonist, angiotensin-ll converting enzyme inhibitor, calcium channel blocker, an 3-hydroxy-3-methylglutaryl-coenzyme (HMG-CoA) reductase inhibitor, digoxin, an anticoagulant, a diuretic, a beta blocker, an angiotensin-converting enzyme (ACE) inhibitor, a steroid, a combination thereof, or the like into the branch, and/or organ and monitoring a physiological response of the subject to the stress test. Such a test may be advantageous for assessing the function of the region, so as for diagnostic purposes, to select one or more regions to ablate, to compare the performance of regions, to assess the suitability of a subject for a therapeutic procedure, etc.

In aspects, the delivery of the bolus may be directed into a lumen of the branch, an adventitia surrounding the branch, into a wall surrounding the lumen, and/or into an organ coupled thereto.

In aspects, the step of delivery may be performed by a delivery system in accordance with the present disclosure. In aspects, the method may include positioning at least a portion of the delivery system into the arterial tree via a main artery serving the tree. In aspects, one or more portions of the delivery system may be embodied within a syringe with a needle in accordance with the present disclosure.

In aspects, the syringe with a needle may be equipped with a substance eluting element, configured to deliver the composition, a substance, a medicament, a denervating substance, a combination thereof, or the like into the target organ, into a perivascular site surrounding the wall of the lumen, into the adventitia of the lumen, into a microenvironment of the tumor, into the lumen, into the tissues surrounding the wall of the lumen, in a region within the wall of the lumen, a combination thereof, or the like.

In aspects, not covered by the scope of the claims, the method may include treating and/or ablating one or more nerves coupled to the region, while substantially limiting damage to the tissues surrounding the region or the nerves, substantially limiting damage to the organ coupled to the region, substantially limiting local inflammation, or the like.

In aspects, not covered by the scope of the claims, induced necrosis will typically cause the corresponding cells to exhibit rapid swelling, lose membrane integrity, shut down metabolism, and release their contents into the environment. Cells that undergo rapid necrosis in vitro do not often have sufficient time or energy to activate apoptotic machinery and thus will often not express apoptotic markers. Rather induced apoptosis typically causes the corresponding cells to exhibit cytological and molecular events such as a change in the refractive index of the cell, cytoplasmic shrinkage, nuclear condensation, and cleavage of DNA (deoxyribonucleic acid) into regularly sized fragments.

In aspects, the composition may be selected so as to induce apoptosis in one or more neural tissues (i.e., axon, dendrite, cell body, myelin sheath, synapse, etc.).

According to aspects, not covered by the scope of the claims, there is provided use of one or more systems, methods, and devices each in accordance with the present disclosure for interventionally altering one or more homeostatic or neuroendocrine processes within a body.

Some non-limiting examples of homeostatic processes include production/release of renin, insulin, cholesterol, bile salts, testosterone, progesterone, prion, serotonin, endorphins, dopamine, monoamine neurotransmitters, histamines, noradrenaline, glucose, and the like, adjustment of blood pressure, anti-inflammatory activity, testosterone, estrogen, "uterine hemorrhaging", hunger, bowel movement, nutritional uptake in the bowel, bone density, a rate of bone remodeling, formation of osteoblasts and the like.

In aspects, a system in accordance with the present disclosure may include a substance delivery aspect, configured for elution of a substance into the vicinity of the target.

### BRIEF DESCRIPTION OF THE DRAWINGS

Several aspects of the disclosure can be better understood with reference to the following drawings. In the drawings, like reference numerals designate corresponding parts throughout the several views.
Figs. 1a1b show an example of tissue ablation with neat ethanol and with a composition in accordance with the present disclosure.
Figs. 2a-2d show schematics of aspects of a delivery system in accordance with the present disclosure.
Figs. 3a3j show aspects of patterned delivery of a composition in accordance with the present disclosure to a volume of tissue.
Figs. 4a4b show aspects of methods in accordance with the present disclosure.
Figs. 5a5l show aspects of delivery tips in accordance with the present disclosure.
Fig. 6 shows application of a composition, delivery system, and delivery tip each in accordance with the present disclosure to treatment of a carotid body.
Figs. 7a7b show aspects of a delivery system in accordance with the present disclosure for treating tissues along a vessel.
Fig. 8 shows aspects of systems and methods for treating cardiac tissue in accordance with the present disclosure.
Figs. 9a9n show aspects of a delivery system and method for treating tissues in a thin walled structure.
Figs. 10a10b show schematics of aspects of a delivery system and composition for treating a volume of tissues in an organ in a body in accordance with the present disclosure.

### DETAILED DESCRIPTION

Particular embodiments of the present disclosure are described hereinbelow with reference to the accompanying drawings; however, the disclosed embodiments are merely examples of the disclosure and may be embodied in various forms. Therefore, specific structural and functional details disclosed herein are not to be interpreted as limiting, but merely as an illustrative basis for the claims and as a representative basis for teaching one skilled in the art to

According to aspects, there is provided a composition for ablation of a tissue site in a body, the composition including a tissue ablating agent for actively treating the tissues in the vicinity of the tissue site, and an excipient for regulating migration and/or a release rate of the tissue ablating agent away from the tissue site upon injection into the tissue site.

The tissue ablating agent includes ethanol. In aspects, the tissue ablating agent may perform at least a portion of the function of a vehicle for delivery of the composition to the tissue site.

The excipient comprises hydroxypropyl cellulose having an average molecular weight of approximately 1,000,000 or greater, and may further include silica, polyvinylpyrrolidone (PVP), glycerin, polyethylene glycol, chitosan, acelated monoglycerides, glycerides, oil, wax, collagen, bovine collagen, cellulose gum, Contigen^{®}, Duraphere^{®}, polyacrylic acid, polyvinyl alcohol, polyvinyl alcohol copolymer, calcium hydroxylapatite (CaHA), calcium acetate, polymaleic acid, polyvinyl methyl ether, silicone, polydimethylsiloxane, glycosaminoglycans, mucopolysaccharides, hyaluronic acid, hyaluronan, autologous fat, autologous ear chondrocytes, polytetrafluoroethylene, cellulose, combinations, copolymers, derivatives, analogs, tautomeric forms, stereoisomers, polymorphs, solvates, salts, nano/micro particulates, and metabolites thereof, or the like.

In aspects, the excipient may futher include a polysaccharide, a starch, a glucan, a glucose polymer, cellulose, combinations, copolymers, derivatives, analogs, tautomeric forms, stereoisomers, polymorphs, solvates, salts, nano/micro particulates, oxidated forms, esters, ethers, and metabolites thereof, or the like. Some non-limiting examples of cellulose derivatives include ethylcellulose (EC), hydroxypropylcellulose (HPC), methylcellulose (MC), The cellulose may be selected from a group of cellulose derivatives that are at least partially soluble in the ablating agent or a vehicle (e.g., a solvent, dimethyl sulfoxide, ethyl acetate, an alcohol, a processing agent, etc.) included in the composition, and in an aqueous medium (e.g., water, saline, normal saline, hypertonic saline, etc.). In aspects, the cellulose may have a substantially higher solubility in the ablating agent or the vehicle than in the aqueous medium. In aspects, the cellulose derivative may have an ethoxyl content of between 45 - 52%, between 47 - 49.5%, etc.

Some non-limiting examples of starch derivatives include dextrin, acid-modified starch, alkaline-modified starch, bleached starch, oxidized starch, enzyme-treated starch, maltodextrin, cyclodextrin, monostarch phosphate, distarch phosphate, acetylated starch, hydroxypropylated starch, hydroxyethyl starch, starch sodium octenyl succinate (OSA) starch, starch aluminium octenyl succinate, cationic starch, carboxymethylated starch, phosphated distarch phosphate, acetylated distarch phosphate, acetylated distarch adipate, hydroxypropyl distarch phosphate, acetylated oxidized starch, monostarch phosphate, distarch phosphate, phosphated distarch phosphate, acetylated distarch phosphate, starch acetate, acetylated distarch adipate, hydroxypropyl starch, hydroxypropyl distarch phosphate, hydroxypropyl distarch glycerol, combinations, copolymers, derivatives, analogs, tautomeric forms, stereoisomers, polymorphs, solvates, salts, nano/micro particulates, and metabolites thereof, or the like

The composition includes one or more surfactants (e.g., an anionic, nonionic, cationic, amphoteric surfactant, sodium lauryl sulfate, ammonium lauryl sulfate, lauryl alcohol ether sulfate, trimethylcoco ammonium chloride, etc.), the surfactant configured so as to maintain the integrity of the composition over a wider temperature range, pH range, to compatibilize one or more components of the composition with a vehicle, to improve wetting of a tissue interface upon delivery thereto, or the like, than achievable without the surfactant.

In aspects, a thermal stabilizing agent may be added to the composition, such as an organic liquid, a surfactant, an alcohol, an aqueous glycol, or the like. Such thermal stabilizing agent may be advantageous to increase the temperature range over which the composition may remain stable at the tissue site, during storage, during delivery to a tissue site, etc. In aspects, the composition may be thermally stable over a temperature range of 10 - 60°C, 10 - 50°C, 10 - 45°C, or the like. In aspects, the composition may be formulated (e.g., with a cellulose derivative based excipient in accordance with the present disclosure) such that the viscosity of the composition at body temperature (approximately 37°C), is substantially higher than the viscosity in the range of 45 - 50°C. In aspects, the composition is formulated such that the ratio between viscosities between 37°C:50°C is greater than 10:1, greater than 100:1, greater than 1000:1, etc.

In aspects, the composition includes hydroxypropylcellulose (HPC) the thermal viscosity profile of the cellulose derivative and the vehicle including a high viscosity over a first pH range, and a low viscosity over a second pH range. In aspects, the first pH range may be near 7, near 7.4, etc. In aspects, the second pH range may be greater than 7.5, greater than 7.7, less than 5, less than 4, or the like.

In aspects, the composition may include an inorganic salt, a dissolved material, sucrose, glucose, combinations thereof, or the like.

In aspects, the composition may include a defoaming agent, a lauryl alcohol, octyl alcohol, etc.

In aspects, the composition may include a cellular therapeutic agent, a myoblast, a fibroblast, a stem cell (a muscle-derived, or adipose-derived stem cell, etc.), a multipotent hematopoietic stem cell (autogeneic, allogeneic, etc.), or the like. Such cellular therapeutic agents may be delivered to a tissue site in a body within a composition in accordance with the present disclosure so as to precisely retain the cells during the implantation stage into the subject, to prevent widespread migration of the cells into the blood stream, etc.

In aspects, the composition may include a polymerizing agent, a polymer, gelatin, pectin, xanthan gum, polysaccharide, polyvinyl alcohol, poly(lactic-co-glycolic acid) (PLGA), ethylene vinyl alcohol (EvOH), or the like. Such polymer forming agents may be advantageous to form a gelatinous, or solid-like bolus of the composition after delivery to a tissue site in the body.

In aspects, the composition may include a tissue adhesive agent, a tissue glue, a fibrin, a fibrin sealant, fibrinogen, thrombin, a cyanoacrylate, n-butyle-2-cyanoacrylate, combinations, derivatives, analogs, tautomeric forms, stereoisomers, polymorphs, solvates, salts, and metabolites thereof, or the like.

In aspects, the composition may include a contrast agent, a CT contrast agent, an iodine or barium based agent, an ionic iodinated medium, diatrizoate, metrizoate, ioxaglate, a nonionic iodinated medium, iopamidol, iohexol, ioxilan, iopromide, iodixanol, a barium sulfate, an MR contrast agent, a gadinolium based medium, omniscan, prohance, gadavist, optimark, magnevist, dotarem, primovist, an iron oxide based medium, a protein based agent, amino acid bound gadolinium media, combinations thereof, or the like.

In aspects, the composition may be formulated as a highly viscous fluid, or a gel, the composition including an excipient in accordance with the present disclosure, and the tissue ablating agent forming at least a portion of a vehicle for the fluid or gel medium.

In aspects, a composition in accordance with the present disclosure may be configured as a gel, the tissue ablating agent represents more than 85% of the composition by mass.

Such a configuration may be advantageous to augment local neural traffic or to defunctionalize the local nerves without inducing cell death.

In aspects, the composition may be formulated as a non-Newtonian fluid, a shear thinning medium (e.g., a thixotropic medium, a pseudoplastic medium, a Bingham plastic, etc.). In aspects, the composition may be formulated as a Bingham plastic, with a yield strength of greater than 5 Pa, greater than 20 Pa, greater than 100 Pa, or the like. The pseudo gel-like composition may behave as a plastic fluid having a high yield strength, high viscosity, and/or low gel strength. The yield strength may be independent of shear stress, shear rate, total work input, and time under stress. Plastic fluids were defined by Bingham as fluids having a yield strength that must be exceeded in order to initiate flow. In aspects, the yield stress of the pseudo gel-like composition may be configured such that the gel can flow freely through a delivery catheter under a high shear condition, but the flow substantially stops when the force applied is less than the force required to overcome the yield strength, forming essentially a pseudo solid-like gel.

In aspects, the composition may be formulated so as to behave as a thixotropic medium, thus flowing more freely once flowing has been initiated, the medium having a thixotropic index (as measured with a viscometer at two different shear rates, such as a first rate and 10x the first rate, with the same spindle and measurement temperature), of greater than 1.25, greater than 1.5, greater than 2, greater than 4, etc. Such a configuration may be advantageous for delivery of the composition to a tissue site through a delivery system in accordance with the present disclosure, while retaining a high degree of stability after delivery to the tissue site.

In aspects, the composition may be configured so as to exhibit a phase change property dictated by the local environment (e.g., local temperature, pH, humidity, salinity, etc.). The composition may include one or more environmentally, anion-responsive, organogels, or the like. The composition may include a first gelator molecule, configured to form a stable first fluid or gel state in a first solution (e.g., such as in the tissue ablating agent), over a first range of temperatures, pH, salinity, etc., the gelator molecule configured to form a second fluid or gel state over a second range of temperatures, pH, salinity, in the presence of a second solution (e.g., a surrounding aqueous medium, in the presence of an analyte, an enzyme, a protein, or the like). In aspects, the transition between the first fluid or gel state to the second fluid or gel state may be advantageous in expelling the tissue ablating agent, retaining the tissue ablating agent, releasing a medicament into the tissue site, increasing the viscosity or yield stress of the medium upon placement at a tissue site, etc. In aspects, the composition may include an anion-responsive organogel, a benzaldehyde based gelator, etc.

In aspects, the composition may be configured such that at a first temperature or environmental state, the composition has a low viscosity suitable for delivery through an elongate delivery catheter to a deployment site in a body at a second temperature or environmental state (e.g., pH, salinity, analyte presence, concentration, etc.). Upon delivery to the second temperature or environmental state, the composition transitions to a high viscosity state, a gel state, a thixotropic state, etc. so as to be more easily retained at the tissue site. In aspects, a composition including a cellulose derivative in accordance with the present disclosure may be configured such that the viscosity of the composition is less than 100 mPa.s (cPs), less than 25 cPa.s (cPs), less than 5 mPa.s (cPs) in a first temperature range of 45 - 50°C, and has a viscosity of greater than 500 mPa.s (cPs), greater than 2000 mPa.s (cPs), greater than 8000 mPa.s (cPs) in a temperature range of 35 - 40°C. In aspects, a composition including a polysaccharide, a starch, a cellulose, derivatives, combinations, or salts thereof in accordance with the present disclosure may be configured such that the viscosity in a tissue ablating medium in accordance with the present disclosure over a temperature range of 35 - 40°C may be less than 100cps, less than 50cps, less than 5cps, while the viscosity may be greater than 500 mPa.s (cps), greater than 2000 mPa.s (cps), greater than 8000 mPa.s (cps) the presence of an aqueous solution over the same temperature range. Such a configuration may be advantageous for quick delivery to the tissue site, while offering adequate retention at the site once delivered.

In aspects, the step of heating may be used to alter one or more properties of the composition selected from the adhesive tack, stiffness, bioavailability, hydrophilic properties, hydrophobic properties, anti-thrombogenic properties, antibacterial properties, combinations thereof, or the like. Such changes may be advantageous to provide increased flow during delivery, to adjust adhesion to the delivery catheter walls, to alter the affinity of the composition to the walls of the delivery catheter (i.e., such as to reduce the wall adhesion during delivery), to prevent or accelerate thrombogenic properties of the gel during delivery and/or after delivery, etc.

In aspects, the composition may include one or more of a non-reactive powder, gelatin, proteins, polysaccharides, corn starch, cane sugar, brown sugar, a salt, sodium chloride, potassium chloride, baking soda, silica, treated silica, nanoclay, rice flour, wheat flour, confectioners' sugar, combinations thereof, flow facilitating particles, blends, combinations thereof, or the like. Such additives may be used to adjust the flow characteristics of a composition in accordance with the present disclosure, to adjust the glass transition temperature, the viscosity temperature profile, etc.

In aspects, the composition may include one or more of fibers, a reactive specie, a non-reactive specie, colorants, powders, films, particles, dyes, proteins, biomarkers, conductive particles, antibacterial species, a linking molecule, a silane, a siloxane, a mucoadhesive molecule, a hydrophilic polymer, a polyethylene glycol, an isocyanate, poly(ethylene glycol)-adipic acid esters, combinations thereof, or the like.

In aspects, the composition may include a curable adhesive composition wherein the curing or thermosetting reaction occurs after delivery to the tissue site. Some non-limiting examples of curable gel adhesives include silicone gel adhesive, a polyurethane gel adhesive, an acrylic gel adhesive, a hydrogel adhesive, a hydrocolloid adhesive, a hydrogel adhesive, a fibrin adhesive, combinations thereof, and or the like.

According to aspects, there is provided a delivery system comprising a delivery tool including a lumen, the lumen forming a fluid coupling between a distal end and a proximal end of the delivery tool, wherein the delivery tool including a lumen comprises a syringe with a needle; a reservoir for retaining a composition according to claims 1-7 prior to delivery of the composition to a treatment site within a volume of tissue, the reservoir coupled with the proximal end of the delivery tool; an injector coupled to the reservoir, the injector configured to deliver a bolus of the composition into the delivery tool upon activation thereof; and a delivery tip coupled to the lumen, the delivery tip deploy-ably coupled to the delivery tool, shaped and dimensioned so as to penetrate into or bias against the volume of tissue upon deployment from the delivery tool, the delivery tip comprising one or more ports coupled to the lumen, the ports arranged upon the delivery tip so as to access the treatment site.

The delivery system may include a thermally controlled reservoir, coupled to the catheter, the thermally controlled reservoir configured to maintain the composition at a first temperature prior to delivery of the fluid into the lumen of the catheter. In aspects, the thermally controlled reservoir may include a heating/cooling element configured and controlled to maintain the composition at the first temperature (e.g., 40 - 45°C, 45 - 50°C, etc.). In aspects, the reservoir may include an energy delivery element, an ultrasonic delivery element, etc., to agitate the composition prior to delivery, the agitation configured so as to reduce the viscosity thereof prior to delivery into the catheter.

In aspects, the delivery system may include a power injector, a syringe pump, or the like, configured to interact with the reservoir so as to deliver the composition to the tissue site during use.

In aspects, the composition may include a chemotherapeutic agent, a cytotoxic agent, an antibody drug conjugate, an anti-neural growth factor, a mitotic inhibitor, a poison, a neurotoxin, or the like.

In aspects, a composition in accordance with the present disclosure may include a toxic substance, ethanol, a small organic molecule, a protein, an enzyme, an amino acid, a bioactive agent (e.g., cells, matrix, viral vectors, DNA, ribonucleic acid (RNA) etc.), botulinum toxin (e.g., Botox^{®}), cytokines, one or more growth factors, combinations thereof, or the like.

In aspects, the composition may include a spindle-cell poison (DM-1, DM-4, calicheamicin, monomethyl auristatin F & E), adriamycin, irinotecan metabolite SN-38, doxorubicin, a taxel, paclitaxel, docetaxel, combinations thereof, or the like.

In aspects, the composition may include one or more neurotoxins or neuroblockers, such as ethanol, glutamate, nitric oxide, botulinum toxin, tetanus toxin, tetrodotoxin, tetraethylammonium, chlorotoxin, conotoxin, bungarotoxin, anatoxin-a, curare, polybrominated diphenyl ether, isobutronitrile, hexachlorophene, metaldehyde, propoxur, hexane, styrene, bifenthrin, 25I-NBOMe, JWH-018, aluminum, arsenic, ammonia, an NMDA receptor blocker, NSAIDs, an NK-1 receptor blocker, FAAH inhibitor, Na, Ca, K channel modulator (e.g., TRPV1, V3, V4, NaV1.7, NaV1.8, ASIC3, etc.), a cannabinoid receptor blocker (CB1, CB2, etc.), delta opioid agonists, P2X3 inhibitors, P38 kinase, CR845, and the like.

In aspects, the composition may include a nerve blocking agent, a sympathetic nerve blocking agent, a parasympathetic nerve blocking agent, an anticholinergic agent, an antimuscarinic agent, a ganglionic blocker, a neuromuscular blocker, combinations thereof, or the like.

Some non-limiting examples of anticholinergic agents include atropine, benztropine, biperiden, chlorpheniramine, dicyclomine, dimenhydrinate, doxylamine, glycopyrrolate, ipratropium, orphenadrine, oxitropiu, oxybutynin, tolterodine, trihexyphenidyl, scopolamine, solifenacin, tropicamide, bupropion, dextromethorphan, doxacurium, hexamethonium, mecamylamine, tubocurarine, etc.

Some non-limiting examples of cholinergic agents include acetylcholine, bethanechol, carbachol, methacholine, arecoline, nicotine, muscarine, pilocarpine, donepezil, edrophonium, neostigmine, physostigmine, pyridostigmine, rivastigmine, tacrine, caffeine, hyperzine A, echothiophate, isoflurophate, malathion, cisapride, droperidol, domperidone, metoclopramide, risperidone, paliperidone, trazodone, clonidine, methyldopa, propranolol, prazosin, oxymetazoline, and the like.

Some non-limiting examples of beta blockers include alprenolol, bucindolol, carteolol, carvedilol, labetalol, nadolol, oxprenolol, penbutolol, pindolol, propranolol, sotalol, timolol, eucommia, acebutolol, atenolol, betaxolol, bisoprolol, celiprolol, esmolol, metoprolol, nebivolol, butaxamine, ICI-118, ICI-551, SR 59230A, and the like.

Some non-limiting examples of alpha blockers include phenoxybenzamine, phentolaamine, tolazoline, trazodone, antipsychotics, alfuzosin, prazosin, doxazosin, tamsulosin, terazosin, silodosin, atipamezole, idazoxan, mirtazapine, yohimbine, carvedilol, labetalol, and the like.

Some non-limiting examples of antibody drug conjugates include a conjugate of an antibody (e.g., CD30, CD20, CD19, CD74, GPNMB, Ley, PSMA, CD138, CD56, CD70, CA6, CanAng, SLC44A4, CEACAM5, AGS-16, Anti-Cripto, trastuzumab, rituximab, cetuximab, bevicizumab, etc.) with a cytotoxic agent (e.g., spindle-cell poisons (DM-1, DM-4, calicheamicin, monomethyl auristatin F & E, Adriamycin, irinotecan metabolite SN-38, doxorubicin, etc.).

In aspects, the composition may include an anti-nerve growth factor (NGF), anti-NGF monoclonal antibodies, tanezumab, fulranumab, REGN475, etc.

In aspects, the composition may include a cyclic oligosaccharide, a cyclodextrin (alpha, beta, gamma, etc.). The cyclodextrin may house one or more active agents, tissue ablative agents, antibody drug conjugates, anti-nerve growth factor, neurotoxin, nerve growth factor, poison, cytotoxic agent, or the like. After delivery of a bolus of the composition to a tissue site in a body, the cyclodextrin may facilitate delivery of one or more of the housed agents to the surrounding tissues, or a nearby organ, etc.

In aspects, the composition may include one or more kinase inhibitors or a steroid for treating a local inflammatory response. The composition may include an excipient that binds to the kinase inhibitor and/or steroid so as to regulate the release rate thereof into the surrounding tissues.

In aspects, the composition may include a crosslinking agent, a PVP (poly vinyl pyrrolidone), a functionalized PVP, etc., the crosslinking agent configured to crosslink with one or more components (e.g., a cellulose derivative, etc.) of the composition, when it is brought into contact with an aqueous solution.

In aspects, a composition in accordance with the present disclosure may include a toxin, a neurotoxin, paclitaxel, etc. The paclitaxel may interfere with axonal function and neural regrowth in the vicinity of the injection site, thus assisting with the durability of the therapy. In aspects, the composition may incorporate ethyl alcohol (or an alternative ablating agent), in combination with paclitaxel.

In aspects, a composition in accordance with the present disclosure may include one or more of amiodarone, hydralazine, perhexiline, drugs used to fight cancer, cisplatin, docetaxel, paclitaxel, suramin, vincristine, combinations thereof, or the like.

In aspects, a composition in accordance with the present disclosure may include chloroquine, isoniazid (INH), metronidazole (Flagyl), nitrofurantoin, thalidomide, combinations thereof, or the like.

In aspects, a composition in accordance with the present disclosure may include etanercept, infliximab, leflunomide, combinations thereof, or the like.

In aspects, a composition in accordance with the present disclosure may include an analgesic to affect local neural traffic during the delivery process.

In aspects, a composition in accordance with the present disclosure may include one or more of dapsone, an anticonvulsant (phenytoin), an anti-alcohol drug (disulfiram), a combination thereof, or the like.

In aspects, a composition in accordance with the present disclosure may include one or more of didanosine (Videx^{®}), stavudine (Zerit^{®}), zalcitabine (Hivid^{®}), arsenic, colchicine, gold, combinations thereof, or the like.

In aspects, not according to the scope of the claims, a system in accordance with the present disclosure may include a sensory subsystem in accordance with the present disclosure. In aspects, the sensory subsystem may include one or more microelectrodes mounted to the catheter, near the distal tip thereof (i.e., near to the tissue site during a delivery process). The microelectrodes may be configured to capture electrophysiological signals, neural traffic signals, chemical migration margin information, or the like from the delivery site.

In aspects, not according to the scope of the claims, a system in accordance with the present disclosure may include a processor, the processor coupled to the sensory subsystem, or to signals generated therefrom, the processor configured to condition and/or display one or more signals associated with the delivery process (e.g., margin of the delivered bolus, migration of the composition over time, etc.), physiologic changes (e.g., changes in pH, salinity, water content, changes in a systemically measured surrogate marker for the procedure, blood pressure, glucose levels, renin levels, noradrenalin spillover, etc.), electrophysiological changes (e.g., changes in neural traffic, changes in nerve function, changes in one or more nerve signals, changes in the character of nearby action potentials, changes in the phasic character of the action potentials, biphasic to monophasic transitions in such action potentials, etc.).

In aspects, not according to the scope of the claims, the processor may include a function to determine the proportion of signals measured from the nerves associated with group I, group II, group III, and/or group IV nerve types. In aspects, the processor may be configured to deliver energy and/or the substance to the tissues until a significant drop in group IV traffic is determined by the function from one or more of the sensory signals.

In aspects, not according to the scope of the claims, a method in accordance with the present disclosure may include determining the proportion of signals measured from the nerves associated with group I, group II, group III, and/or group IV nerve types, the ablating and/or defunctionalizing dependent upon the proportion. In aspects, the step of ablating and/or defunctionalizing may be adapted so as to stop based upon a substantial drop in group IV traffic (e.g., such as by halting delivery of the substance, by delivering a neutralizing substance, by delivering an antidote, by withdrawing the delivery element, etc.). In aspects, the determination of group traffic may include analyzing the shapes and/or propagation characteristics of action potentials as measured amongst a plurality of electrodes in accordance with the present disclosure.

In aspects, not according to the scope of the claims, the method may include monitoring the extent of effect that a composition has on the group I, group II, or group III traffic as measured near to, or coupled to the tissue site. In aspects, the method may include halting delivery of the composition if the traffic changes are not as desired for the given therapy (i.e., if the changes in group I or group II traffic are sufficiently higher than accepted).

In aspects, not according to the scope of the claims, the method may include ablating and/or defunctionalizing one or more nerves associated with group III or group IV, while substantially preserving one or more nerves associated with group I or group II. Such ablation and/or defunctionalization may be achieved through selection of active substances in a composition in accordance with the present disclosure, and precise delivery and optional monitoring of the effect of the composition to the tissue site in the body.

According to aspects, not according to the scope of the claims, there is provided a system, a composition, and a method each in accordance with the present disclosure for treating one or more classifications of nerves, muscles, and/or receptors at sites within a body to alter a neuroendocrine, neural, or cardiac function thereof. The method includes selecting a composition in accordance with the present disclosure, the composition being selective to the target nerve, muscle, or receptors, delivering the composition to the sites within the body, and optionally monitoring one or more of nerve traffic, a physiologic surrogate parameter related to the nerve traffic, or the like to determine the extent of treatment. The composition may be delivered, and optionally the effects monitored with a system in accordance with the present disclosure.

According to aspects, not according to the scope of the claims, there is provided a method for determining the extent of a treatment at a site within a body, the method including administering a composition in accordance with the present disclosure to the site, and monitoring a change in neural traffic in the vicinity of the site, the neural traffic changing with the extent of the treatment, and analyzing the change in neural traffic to determine if the treatment is substantially complete. In aspects, the analyzing may include analyzing one or more action potentials in the neural traffic to determine the type of nerves affected by the treatment, analyzing the action potentials to determine a change in spectral composition thereof as effected by the treatment, analyzing the propagation velocity of one or more action potentials to determine the extent of the change therein as caused by the treatment.

The step of analyzing the action potentials not according to the scope of the claims, may include analyzing a change in the rise time of the action potential, a change in the pulse width of the action potential, a change in the spectral content of the action potential, a change in the periodicity of similar action potentials (as measured at one or more monitoring sites around the treatment site), a change in the number of similar action potentials per unit of time, a change in the polarity of action potentials (e.g., a change in the number or percentage of positive polarity action potentials, a change in the number or percentage of negative polarity action potentials, a change in the polarity of the aggregate traffic measurement, etc.).

In aspects, the composition may be configured to form at least a partial collagen block at the delivery site. Such a collagen block may be formed by healing of tissues after a sufficiently disruptive ablation event, caused by an overexpression of a scar growth factor, caused by prolonged healing and inflammatory response around one or more constituents in the composition (such as an ink, a contrast agent, a filler, a silica micro or nano particle, etc.). Such formation of a fibrotic or collagen block may be advantageous to limit nerve regrowth after the treatment, to block cell migration along a pre-existing neural pathway, etc.

In aspects, the composition may include a poison, neurotoxin, or anti-nerve growth factor, configured to down regulate local nerve growth and/or limit local nerve regrowth at the delivery site. In aspects, the composition may include an anti-nerve growth factor, a microtubule disruptor, paclitaxel, or the like to limit nerve regrowth and/or neural sprouting in the vicinity of the delivery site. Such an approach may be advantageous to limit neuritis (nerve regrowth with heightened pain, often perceived as worse than before the surgery, which can occur during pain management treatments), anesthesia dolorosa (patient complaints of distressing numbness), and side effects associated with poorly controlled treatments (e.g., such as may be caused by migration of prior art therapeutic agents).

In aspects, the composition may include a polymer, a precipitating component, and/or a gelating agent in accordance with the present disclosure. Such a polymer, precipitating component, or gelating agent may be configured to form a skin around a bolus of the composition after delivery to a treatment site. The skin may be configured with a permeability configured to provide a slow leakage of an active agent (e.g., a tissue ablating agent, an anti-nerve growth factor, a nerve growth factor, a toxic substance, a poison, a neurotoxin, etc.) into the surrounding tissues for a period of time following the delivery of the bolus thereto.

In aspects, the skin forming component may be biodegradable, metabolizable (e.g., a sugar, a carbohydrate, sucrose, a fatty acid, a starch, etc.), etc.

The composition in accordance with the present disclosure includes hydroxypropylcellulose. Upon delivery of the composition to a tissue site in a body, the cellulose derivative may form a skin around the bolus, thus creating a diffusion barrier. In aspects, the hydroxypropylcellulose may be configured so as to readily breakdown and metabolize in the body, such that only a temporary barrier is formed upon injection of the composition.

In aspects, the polymer, precipitating component, and/or gelating agent may be configured to form a substantially strong barrier in the presence of a first medium (e.g., blood, urine, air, lymph, bile, etc.), and a substantially weak barrier in the presence of a second medium (e.g., interstitial fluid, extracellular fluid, water, fatty tissue, etc.), such that release of the active agent is provided towards the second medium. In aspects, a cellulose derivative in accordance with the present disclosure may be configured to form a plug in the presence of a first medium (e.g., blood), and to remain within the solution of the composition in the presence of the second medium (e.g., interstitial fluid). Such a configuration may be advantageous to limit flashback along an injection pathway, to limit migration of the composition into a nearby blood vessel, etc.

In aspects, a delivery system in accordance with the present disclosure may include a hollow stem delivery tube configured for placement into the wall of a vessel, and a composition, configured to form a sack-like bolus after passage through the stem, the delivery system configured to pierce the stem through and embed the stem into the wall of a lumen, the sack-like bolus to form a fluid reservoir on the other side thereof. After placement, the composition may slowly transfer from the fluid reservoir, through the hollow stem, and into the vessel. Such a configuration may be advantageous to slowly release an active agent into a vessel within a body.

In aspects, not according to the scope of the claims, the delivery system may include an anchor, configured for placement into the wall, the anchor coupled to the hollow stem delivery tube, the hollow stem and/or the anchor providing fluid communication between the fluid reservoir and the vessel.

In aspects, not according to the scope of the claims, the hollow stem, anchor, or the like may be biodegradable. The hollow stem, anchor, or the like may be formed from a biodegradable polymer (e.g., polylactic acid (PLA), PLGA, polysaccharides, collagen, etc.), a magnesium or potassium based structure, or the like.

The delivery system is configured (such as via shape, composition, permeability, etc.) so as to slowly release a pattern of a medicament into a tissue, organ, lumen wall, etc. in the body.

The composition in accordance with the present disclosure may be used to treat one or more of ablation, of a tissue or any other biological tissue present at a delivery site. The composition may be formulated so as to control the rate of release, migration, retain treatment at a delivery site, etc.

In aspects, the composition may be configured to form a complete ablation of adjacent tissues, present at the delivery site.

The composition may be biostable or bioerodable, biocompatible with minimal toxicity to surrounding tissues except for the targeted tissue type, configured so as to cause an inflammatory or otherwise cytotoxic response upon delivery.

The composition is configured so as to substantially minimize migration upon delivery to a tissue site in a body. An associated delivery system in accordance with the present disclosure may be configured to be laid down, to inject, etc. a composition in accordance with the present disclosure in one or more physical forms, configurations, sizes, or shapes on biological surfaces or within a three dimensional volume of tissue (e.g., to form a ring, a fence, a wall, to shape electrophysiological signal traffic throughout the volume of tissue, to target specific sites within the volume of tissue, to isolate a region of the tissue, etc.).

The delivery system in accordance with the present disclosure includes a needle, through which a composition may be delivered to a tissue surface, or volume, the needle shaped, and configured to shape the composition (e.g., as a spherical shape, a line, a ring, along a pathway, a fence, bell shapes, elliptical shapes, etc.). In aspects, the needle may include one or more ports through which a composition may be delivered.

According to aspects, there is provided an injection device for delivering a composition in accordance with the present disclosure to one or more tissue sites in a body, the injection device including a needle, the needle including one or more lumens for delivering the composition. The needle may be configured with an occluded tip, or an open tip, may include one or more ports along a wall thereof, may be shaped so as to pattern the composition into a shaped pattern along a tissue surface, or into a three dimensional volume of tissue, shaped so as to adjust an injection rate, size, shape, dose, or distribution of the pattern, etc.

Such a configuration may be used to control a pattern of injection: spherical, linear, ellipsoidal, or other two-dimensional/three-dimensional shape, which may be advantageous for treating a tissue, a region of tissue, a pattern of tissue along a wall, to deliver a medicament to a specific site along a wall of an organ, through a vessel, into a region of tissue beyond a vessel, along a region of muscle, to isolate a region of muscle, to treat a neuromuscular interface, etc.

In aspects, a delivery system/an injection device in accordance with the present disclosure may include one or more sensing components, the sensing components configured to monitor one or more of neural activity, autonomic nervous system activity, afferent nerve traffic, efferent nerve traffic, sympathetic nerve traffic, parasympathetic nerve traffic, electromyographic activity, smooth muscle activity, cardiac muscle electrophysiological activity, intracardiac activity (myopotentials, His-Purkinje pathways), transition between different types of tissue (e.g., such as by impedance measurement, local stiffness measurement, light scatter measurement, etc.), combinations thereof, or the like. In aspects, the sensing components may include one or more electrodes, each electrode configured to sample the activity locally around the tip of an injection device, near to an injection site to determine the margins of the effect of the injection, at a remote site to determine the effect of a delivered composition, etc. One or more of the sensing components may be applied along a needle, a plurality of sensing components may be patterned along and around the needle, etc. In aspects, a plurality of sensing components may be applied along a length of a needle, the sensing components coupled with microelectronics so as to measure impedance, Nernst potentials, biopotentials, etc. there between. Such microelectronics may be configured to determine when one or more sensing components have passed into a lumen wall, is in contact with a fluid (such as blood), has passed from a first tissue type, into a second tissue type, etc. Such information may be used to help guide the needle towards a target site, to determine if the needle tip has left the lumen through which it has been guided to the target site, if the needle tip has been guided to a target neural structure, etc.

In aspects, a composition in accordance with the present disclosure may be configured to deliver a matrix of a tissue ablating agent into a volume of tissue. In aspects, the composition may be configured as an electrically insulating composition, the sensing component configured to determine the margins of the bolus (e.g., via monitoring conductivity between two or more electrodes in the vicinity of the delivery site, etc.).

In aspects, a composition in accordance with the present disclosure may include one or more electronic or ionic conducting components (e.g., a conjugated polymer, a salt, a conducting composite, etc.). In aspects, the composition may be configured such that the electronic or ionic conducting component may be polymerized in place after delivery to a treatment site, may be configured so as to interrupt local electrophysiological processes (e.g., interrupt signal traffic through a volume of cardiac tissue, along a nerve plexus, etc.). In aspects, the conducting component may be electropolymerized in place, using one or more electrodes in close proximity thereto, and/or a remote return electrode placed elsewhere on or in the body.

In aspects, the composition may be configured so as to limit migration from an injection site to a distance of less than approximately 3mm, less than approximately 2mm, less than approximately 1mm, etc. In aspects, the composition may be formulated such that the migration is sufficient so as to link adjacently placed boluses, but not so much so as to limit collateral damage during the treatment process. In aspects, the composition may include one or more contrast agents (e.g., a radiological contrast agent, an ultrasound contrast agent, a MR contrast agent, a fluoroscopic contrast agent, etc.) in accordance with the present disclosure, such that the placement and/or migration of the boluses may be visualized during a procedure.

In aspects, the procedure may be used to treat one or more sites along an organ wall (e.g., a bladder, a urethra, a ureter, a prostate, a testicle, a heart, a liver, a stomach, a bowel, a biliary tract, a pancreas, a kidney, an artery, a vein, a vessel, a lymph node, a bone, a periosteal space, a lung, a bronchial tract, a gland, a ganglion, a region of the limbic brain, an ovary, a uterus, etc.). In aspects, the composition may include a contrast agent in accordance with the present disclosure, such that an operator may visualize where the composition has been delivered along the organ wall, where it has migrated to, etc.

In aspects, a composition in accordance with the present disclosure may include a salt, a hypertonic solution, or the like.

In aspects, a sensory component in accordance with the present disclosure may be used to determine the ischemic border zones/the isthmus for ischemic myocardium using one or more sensors on the tip of a delivery system or injection device in accordance with the present disclosure. Once the border zone is detected, the delivery system or injection device may deliver one or more boluses of a composition in accordance with the present disclosure to treat the border. Optionally, the sensory component may be configured to monitor the effect of the composition on the electrophysiological activity along the border, so as to determine when the treatment has been completed.

In aspects, the composition may be configured to perform a cryoablative procedure on tissues in the vicinity thereof (i.e., by delivery of a super-cooled composition, a composition for providing a localized endothermic reaction, etc.). In aspects, such cryoablative compositions may include one or more metal complexes, a metal complex in combination with a salt solution, etc. In aspects, the composition may be configured as a two part solution, the two parts mixed before, during, and/or after delivery to the tissue site.

In aspects, a composition in accordance with the present disclosure may include a phase change component, such as a polymerizing element, a gel forming element, a gelling agent, an ion exchange gel, etc. In aspects, the phase change component may be configured as follows. The composition may be delivered to the tissue site as a fluid, the fluid surrounding a neural structure of interest. Upon delivery, the phase change component of the composition transitions to a gel state, a polymerization reaction takes place, etc. and the phase change component transitions into a substantially solid mass, effectively surrounding the neural structure of interest (e.g., a ganglion, a nerve plexus, etc.). In aspects, the composition may include a hypertonic or hypotonic solution, a solvent, etc. such that exchange of the solution or solvent with the surroundings results in a net shrinkage of the substantially solid mass after placement around the neural structure. Such shrinkage may effectively compress the neural structure, thereby instilling a neural block thereto (i.e., effectively blocking traffic along the neural structures while otherwise minimizing necrosis and cell death of the neural structures). Such a configuration may be advantageous for affecting neurological function at a tissue site while minimizing associated nerve growth, which may occur in response to local inflammation, damage to the nerves, etc.

In aspects, the composition may include a gelling agent such as a hydrophilic polymer, a free radical forming component, a crosslinking polymer system, a 2 part gel system, or the like. In aspects, a delivery system in accordance with the present disclosure may include a mixing element, a static mixer, etc. in order to mix the parts prior to or during delivery to a tissue site in the body.

In aspects, a composition, a delivery system, or a method each in accordance with the present disclosure may be applied to treatment of several tissues or disease states within a body, such as the gastrointestinal system, the cardiac system, the neuroendocrine system, the renal system, the ANS (autonomic nervous system), the CNS (central nervous system), a peripheral nerve, a neuromuscular junction, a cancerous tumor, a cosmetic procedure (i.e., combined botox and bulking applications, etc.), and the like.

Some non-limiting examples of treatments for the gastrointestinal system include, treatment of an electrical storm in a bowel, treatment of an autoimmune disorder, treatment of LUTS, overactive bladder (e.g., treatment of receptors in the bladder muscle, in the neural pathway between the bladder and local ganglia, along a muscle wall of a urethra, etc.), incontinence (e.g., urinary or fecal incontinence, adjustment of sphincter tone, etc.), treatment of ulcerations (e.g., via injection of growth factors, topical application thereof, etc.), or the like.

Some non-limiting examples of cardiac applications are for the treatment of atrial arrhythmias (atrial fibrillation (AFib), supraventricular tachycardia (SVT), atrial premature complexes (APCs), atrioventricular nodal reentrant tachycardia (AVNRT), Wolff-Parkinson-White (WPW)/Accessory tract, atrioventricular node (AVN) Ablation), treatment of aFib in specific patterns (e.g., 'dots' or spherical patterns, linear patterns (two-dimensional or three-dimensional shapes), combined with contrast agent to visualize the injected pattern under fluoroscopy, x-ray, MR, or ultrasound-based imaging technologies, etc.). In MR applications, the composition may include one or more ferromagnetic components (e.g., an iron or iron oxide complex, a gadolinium complex, etc.), configured to assist with visualization of the placement of composition into a tissue site, etc.

Such applications may be further improved with combination of a sensing component in accordance with the present disclosure to assess/avoid regions of the esophagus (for example, induce a swallow and sense esophageal EMG (electromyography) within the heart wall prior to injection, to ensure adequate margins, etc.).

Some additional cardiac applications include treatment of ventricular arrhythmias (ventricular tachycardia (VT), ventricular fibrillation (VF), premature ventricular contractions (PVCs)), such as may be accomplished by sensing regions of slowed conduction and ablate selectively with a composition in accordance with the present disclosure, follow this region with further sensing to ablate the entire affected zone. Such treatments may be enhanced with combination of a composition in accordance with the present disclosure and a sensory component in accordance with the present disclosure (such as may be unipolar, bipolar, multipolar, etc. configured to determine epicardial activity during treatment, to determine the extent of the composition treatment, to assist with determining the next site to treat, etc.).

Some additional cardiac applications include treatment of one or more autonomic plexi in the vicinity of the heart or coupled thereto. Such structures related to aFib and other arrhythmogenic foci that are autonomic dependent include ganglia, vagal (hypervagotonia, etc.) and dysautonomias, postural orthostatic tachycardia syndrome (POTS), etc. Such structures may be targeted along/near a vein of Marshall, along the epicardium, along the pericardium, etc.

Some non-limiting applications related to neuroendocrine remodulation include renal nerve treatments, renal artery treatment, treatment of renal accessory vessels, adrenal arteries, carotid sinus, carotid body, autonomic ganglia (e.g., celiac, carotid, etc.), and the like.

Some additional non-limiting applications include treatment of one or more neuroendocrine aspects of congestive heart failure, hypertension, metabolic syndrome (MSx), hypogonadism, inflammatory diseases, infiltrative diseases, infection, chronic wounds, Sjogren's syndrome, Gaucher disease, Parkinson's disease, epilepsy, depression, tumors, stroke, diabetes, cancer, pancreatitis, islet cell tumors, nephrotic syndrome, kidney stones, lower urinary tract disorders, urinary incontinence, urinary tract infection, neurogenic bladder disorders, male or female fertility, impotence, premature ejaculation, prostate cancer, ovary cancer, uterine cancer, gastrointestinal ulcers, acid reflux disorders, celiac disease, irritable bowel syndrome, gastrointestinal cancers, tuberculosis, cystic fibrosis, pulmonary hypertension, chronic obstructive pulmonary disease, lung cancer, coronary artery disease, arrhythmias, and chronic renal failure. Treatment of abnormalities of hormonal secretion such as increased catecholamine, renine and angiotensin II levels, increased blood pressure due to peripheral vascular constriction and/or water and sodium retention, renal failure due to impaired glomerular filtration and nephron loss, cardiac dysfunction and heart failure due to left ventricular hypertrophy and myocyte loss, stroke, and diabetes. Additional treatments may include augmentation of function or a disease state associated with a vessel, an artery, a vein, a tubule, a renal artery, an arteriole, a venule, a duct, a chamber, a pocket, a tubule, a bowel, a urethra, an organ, a combination thereof, or the like.

In aspects, treatment or alteration of function of one or more organs some non-limiting examples including a kidney, a prostate, a testicle, a pancreas, a liver, a lung, a bowel wall, a stomach wall, a gland, a neural body, a carotid body, a gall bladder, a small intestine, a large intestine, a spleen, a pancreas, a bladder, an adrenal gland, a uterus, lymph node, a ganglion, combinations thereof, and the like. Treatment of one or more symptoms, neurological, and/or neuroendocrine contributions to lower urinary tract symptoms (LUTS) secondary to benign prostatic hyperplasia (BPH), chronic prostatitis (CP), hypogonadism (HG), nocturia, prostate cancer (PrCa), and erectile dysfunction (ED), micturition, incontinence, frequency, pain, bladder capacity, and/or configured to modulate neural activity in at least a portion of the bladder wall, or the like.

Such compositions, delivery systems, and/or methods in accordance with the present disclosure may be used in treatment so as to affect the growth rate, hormone secretion rates, or development of an organ (e.g., a prostate, a testicle, etc.), or a tumor (e.g., a prostate cancer tumor, a perineural invading cancerous tumor, lymphatic invading tumors, etc.), lymphatic ducts, lymphatic nodes, or the like, to alter functions including a sensation (e.g., a hunger sensation, an urge to urinate, pain, etc.), a tremor, altering release/secretion of a chemical substance (e.g., acid, hormones, toxins, bile, enzymes, surfactants, sebum, renin, etc. from a secretory cell), altering smooth muscle tone, or the like. Such a composition, system, or method may be used to treat a disease of the gall bladder, renal system, metabolic functions, gastrointestinal function, to augment hunger sensation, reduce tone, combinations thereof, and the like.

In aspects, some non-limiting examples of medical conditions that can be treated according to the present disclosure include genetic, skeletal, immunological, vascular or hematological, muscular or connective tissue, neurological, ocular, auditory or vestibular, dermatological, endocrinological, olfactory, cardiovascular, genitourinary, psychological, gastrointestinal, respiratory/pulmonary, neoplastic, or inflammatory medical conditions. Further, the medical condition can be the result of any etiology including vascular, ischemic, thrombotic, embolic, infectious (including bacterial, viral, parasitic, fungal, abscessal), neoplastic, drug-induced, metabolic, immunological, collagenic, traumatic, surgical, idiopathic, endocrinological, allergic, degenerative, congenital, or abnormal malformational causes.

The present systems and methods also encompass enhancing the therapeutic effects of other therapies, such as methods and systems working in conjunction with a pharmaceutical agent or other therapies to augment, enhance, improve, or facilitate other therapies (adjunctive therapies) as well as reducing/minimizing and counteracting side effects, complications and adverse reactions for any therapies involved in treating the above-mentioned medical conditions.

In aspects, liver function may be augmented by a treatment and/or monitored in accordance with the present disclosure including glucose storage/release, metabolic sensing (and related signal traffic to the brain related thereto), glucoregulatory function, afferent vagal activity reaching the brain, chemoreceptor function (or related signal traffic associated therewith), lipid sensing/synthesis, regulation of hepatic insulin sensitizing substance, afferent traffic augmentation associated with glucosensors (i.e., primarily in the region of the portal vein, etc.), protein sensing, GLP-1, leptin, CCK, FFA, PPAR alpha and gamma, glycogenolysis, gluconeogenesis, VLDL secretion, ketogenesis, hypoglucemia sensing, or the like.

In aspects, one or more compositions, delivery systems, and/or methods in accordance with the present disclosure may be used to treat cancer of the prostate, pancreas, breast, cervix, ovaries, bladder, bone, combinations thereof, pain associated therewith, or the like. Such applications may include delivery of compositions to slow, to reverse, and/or to prevent perineural and/or lymphatic vessel invasion of a cancerous tumor into a surrounding neural and/or lymphatic microenvironment, to interrupt, decrease, and/or stop neural communication to/from a cancerous tumor and/or the microenvironment surrounding the tumor to a remote site within a body, etc.

In aspects, one or more systems, methods, or compositions in accordance with the present disclosure may be used to treat one or more conditions of the central nervous system, the enteric nervous system, the limbic brain, etc. Some non-limiting examples include treatment of seizure foci, hyperactive neurological regions, neuroendocrine/gastrointestinal (GI) structures, pancreas/b-islet cells for DM, production of ghrelin and other GI hormones, combinations thereof, or the like.

In aspects, one or more non-limiting applications in oncology include sensing and ablation of CNS tumors with chronic release (e.g., CNS tumor with absence of electrical signals indicative of a tumor region, methods of determining and treating tumor border/margin, etc.).

In aspects, one or more non-limiting cosmetic applications include the combination of neurotoxic function with a filler, chronic release of a neurotoxin (e.g., release of botulinum toxin, etc.), combination of bulking agents with neurotoxins (e.g., for treatment of sphincter spasm, sphincter bulking, wrinkle removal, etc.).

In aspects, a delivery system comprising a delivery tool including a lumen, the lumen forming a fluid coupling between a distal end and a proximal end of the delivery tool, wherein the delivery tool including a lumen comprises a syringe with a needle.

In aspects, a system, device, and/or method in accordance with the present disclosure may be used to treat and/or slow the progression of a cancerous tumor. Some non-limiting examples of such cancer that may be treated include cancer of the prostate, pancreas, breast, colon, skin, liver, esophagus, cervix, bone, urogenitals, lung, and the like. In aspects, the progression may be slowed by blocking of neural and/or lymphatic pathways as may otherwise provide conduits for metastasizing tumor cells.

In aspects, a system, device, and/or method in accordance with the present disclosure may be used to slow, hinder, and/or prevent perineural or peri-lymphatic invasion of a cancerous tumor into a surrounding nerve or lymphatic structure.

In aspects, a system, device, and/or method in accordance with the present disclosure may be used to interrupt, decrease, and/or stop neural communication to a cancerous tumor and/or the microenvironment surrounding the tumor (i.e., to interrupt nerve traffic to/from a cancerous tumor or the tissues thereby to the rest of the body).

In aspects, a system, device, and/or method in accordance with the present disclosure may be used to decrease pain signals communicated by nerves in the vicinity of the organ and/or tumor to one or more neural circuits, ganglia, etc.

In aspects, a system, device, and/or method in accordance with the present disclosure may be used to block, deaden, and/or to destroy nerves in the vicinity of a tumor and/or surrounding tissues.

In aspects, a system, device, and/or method in accordance with the present disclosure may be used to slow or even halt tumorigenesis of cancerous tissue.

In aspects, a composition and/or delivery method in accordance with the present disclosure may be configured to form a physical barrier (i.e., lesion, a collagen block, etc.) along a neural structure and/or a lymphatic structure in a body.

In aspects, the composition may include an antibody drug conjugate (ADC), a chemotherapeutic agent, a toxin, a neurotoxin, etc. In aspects, the ADC may be configured to affect the function of a region or tissue type within the vicinity of the organ alternatively to the other tissues within the vicinity thereof. In aspects, the composition may include a sugar attached to a therapeutic agent to mask the therapeutic agent, such that it is to be taken up by the region of tissue (i.e., appear as a sugar, a friendly protein, etc.). Such a configuration provides a method for delivering a highly potent medicament directly to a tissue of interest (i.e., directly into a tumor), so as to enhance the bioavailability thereof, and to minimize the systemic dosage required in order to achieve significant therapeutic concentrations thereof within the region of tissue.

In aspects, the composition may be delivered at a rate of less than 1 milligram/second (mg/sec), 1 milligram/minute (mg/min), 1 milligram/hour (mg/hr), 0.01mg/hr, less than 1 microgram/hour (µg/hr), or the like. Such a configuration may be important so as to minimize local stress and damage caused by the introduction of the composition into the microenvironment of the tissue of interest.

In aspects, the composition may be formulated such that the ablative agent is released from a delivered bolus (e.g., such as a 100mg bolus) into the surrounding tissues at a rate of less than 500mg/sec, less than 50mg/sec, less than 500mg/min, less than 100µg/hr, or the like. In aspects, a slow release formulation may be used so as to functionally disable a tissue site in a body without causing local cell death. Such a configuration may be advantageous for performing a substantially durable and reversible treatment of tissues in a body. In aspects, an active agent may include a phenol, an alcohol, etc. and the composition may include a metabolically cleavable bond (e.g., a sugar, a cellulose chain, etc.) to which the active agent may be bound. Such slow metabolic cleavage of the bonds may allow for exceptionally slow release of the active agent into the surrounding tissues. Such a configuration may be advantageous to control ethanol elution in time and space near to a target tissue site in a body over a period of seconds, minutes, hours, days, weeks, or even longer.

In aspects, a delivery system in accordance with the present disclosure includes a syringe with a needle configured for percutaneous access to the arteries, veins, or lumens, of a body, for delivery through one or more arteries of the body to the vicinity of the target organ.

In aspects, one or more energy delivery elements, sensing elements, a diameter of the catheter, guidewire, or the like may be sized and arranged such that it may be placed within an artery, vein in a region near the target organ, within the parenchyma of the target organ, into a vessel in the periosteal space of a bone, and/or through a foramen of a bone. In aspects, the delivery elements and/or sensing elements, catheter, guidewire, etc. may be sized and dimensioned such that a characteristic diameter thereof is less than 2mm, less than 1mm, less than 0.75mm, less than 0.5mm, less than 0.3mm, or the like.

In aspects, a method in accordance with the present disclosure may be used to treat prostate cancer, pancreatic cancer, breast cancer, colon cancer, cervical cancer, ovarian cancer, bladder cancer, bone cancer, or the like.

In aspects, a system in accordance with the present disclosure may include a substance delivery aspect, configured for elution of a substance into the vicinity of the target.

In aspects, the micro-tool tip may include a substance delivery needle for providing a drug substance to one or more of the nerves to perform the ablation.

In aspects, the micro-tool tip may include an energy delivery means for providing an ablating current, ultrasound energy, high intensity focused ultrasound (HIFU), MR guided HIFU, thermal energy, cryogenic change, etc. to one or more of the nerves.

In aspects, the delivery system may include a signal conditioning circuit and a processor for identifying the presence and/or characterizing one or more of the nerves, to generate a feedback signal therefrom, and to coordinate the energy or substance delivery based upon the feedback signal.

In aspects, the micro-tool tip may have a characteristic diameter of less than 2mm, less than 1mm, less than 0.5mm, less than 0.25mm, or the like to facilitate placement into the vessel.

In aspects, the micro-tool tip may include one or more electrodes in accordance with the present disclosure. One or more of the electrodes may be sized and dimensioned to measure the signal, and/or one or more of the electrodes may be sized and dimensioned to stimulate and/or ablate one or more of the nerves.

In aspects, the micro-tool tip may include a plurality of electrodes, each electrode configured for sensing an electrophysiological signal in accordance with the present disclosure in the vicinity thereof, the electrodes electrically isolated from each other such that the collection of locally collected signals may be used to determine activity over a region of tissues in the vicinity of the vessel.

### Examples and Figures

The compositions, delivery systems, and methods outlined in the in the present disclosure will be better understood by reference to the following examples and Figures, which are offered by way of illustration and which one of skill in the art will recognize are not meant to be limiting.

### Example 1 CONTROL:

A composition of ethyl alcohol (purchased from Sigma Aldrich), was mixed with 0.01% weight (wt) of a fluorescein fluorescent marker. The composition was mixed until a substantially homogenous distribution of the marker was obtained in the solution. This solution was used as a control in the following tests.

### Example 2:

A composition in accordance with the present disclosure was fabricated according to the following recipe. Ethyl alcohol and hydroxypropyl cellulose (HPC) (average Mw of approximately 1 million) were purchased from Sigma Aldrich. 2 parts of the HPC powder were dispersed into 100 parts of ethyl alcohol and mixed with a high shear mixer at a temperature of approximately 45 - 50°C until a substantially homogenous mixture was produced. A fluorescein marker (0.01% by wt) was added to the mixture to assist with visualizing the migration thereof in tissues.

A composition with a low shear rate viscosity of greater than 1000 mPa.s (cPs) was formed.

The resulting composition was loaded into a 0.5mL syringe and was delivered to tissues through a 25 gauge needle in the following tests.

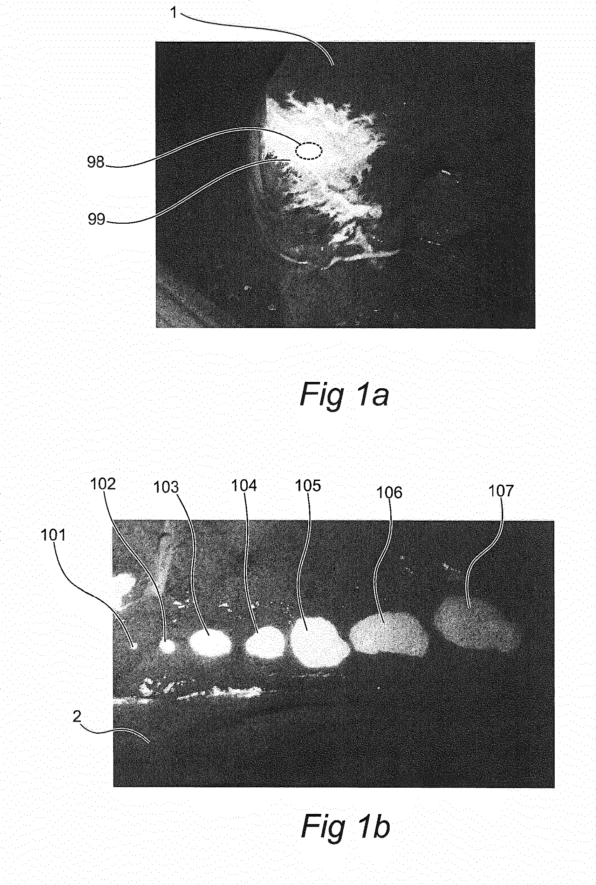

Figs. 1a1b show an example of tissue ablation with neat ethanol (as formulated in Example 1 CONTROL above) and with a composition (as formulated in Example 2 above) in accordance with the present disclosure. Fig. 1a shows the free surface migration of a 50µL bolus 99 of neat ethanol (including a fluorescein fluorescent marker), injected onto a free surface of liver tissue 1. The target ablation zone 98 is highlighted for reference. As shown in Fig. 1a, the ethanol migrated a substantial distance from the delivery site (measured in excess of 30mm from the delivery site), with a substantial portion of the bolus flowing away from the delivery site off of the liver tissues 1. Furthermore, histological analysis of the liver tissue 1 demonstrated very little to the tissue was ablated by the ethanol, with only a small grouping of uncontrolled regions around the deposition site being suitably treated by the bolus. In addition, controlled delivery of a specific bolus of ethanol was challenging given the low viscosity thereof.

Fig. 1b shows free surface migration of a range of bolus sizes of a composition in accordance with the present disclosure, as fabricated in Example 2 on a surface of liver tissue 2. The bolus sizes from left to right are 10 micro liter (µL) 101, 20µL 102, 40µL 103, 40µL 104, 60µL 105, 80µL 106, and 100µL 107. For reference of scale, the 100µL 107 bolus has a total width of approximately 12mm. Histological analysis of the liver sample 2 demonstrated clear, spatially-controlled ablation of tissues under each of the boluses with very clearly defined margins (within 1mm of the fluorescing margins visible in Fig. 1b).

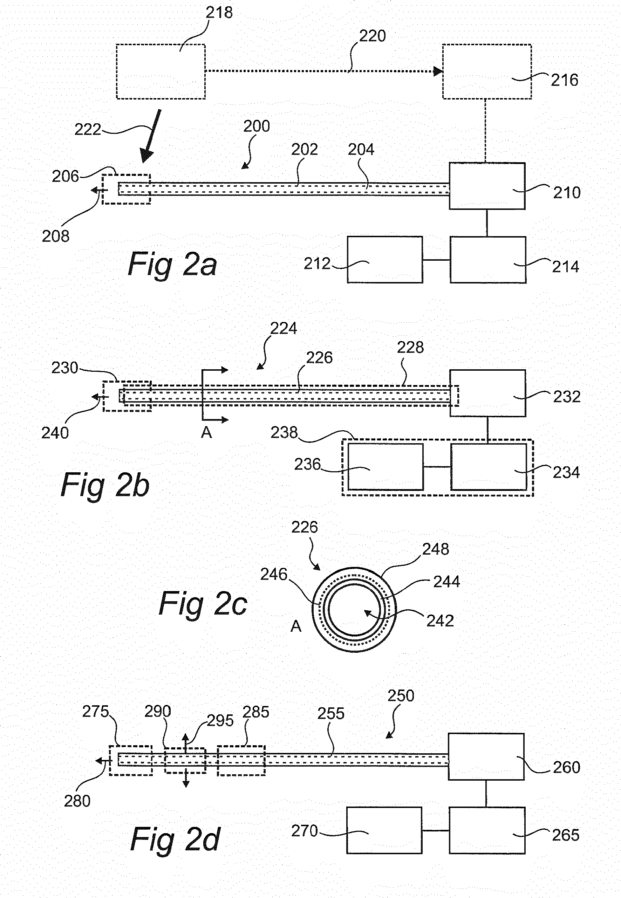

Figs. 2a2d show schematics of aspects of a delivery system in accordance with the present disclosure. Fig. 2a shows aspects of a system for performing a procedure in accordance with the present disclosure. The system is shown as configured for interfacing with a surgical site within a body, a subject, a patient, etc. The system includes a delivery tool 200 in accordance with the present disclosure. The delivery tool 200 may include one or more lumens 204 configured to connect a distal tip thereof to a proximal end (e.g., a controller, a connector, a delivery end, etc.), the lumen 204 shaped and dimensioned such that a composition in accordance with the present disclosure may be delivered 208 to a target site 206 in the body. During use, the delivery tool 200 may be configured to interact with the target site 206 in accordance with the present disclosure. In aspects, the delivery tool 200 may be coupled to a connector 210, the connector providing a mechanical, electrical, fluid, and/or optical interface between the delivery tool 200 and one or more other modules of the system. In aspects, the delivery tool 200 may include an embedded local microcircuit (a microcircuit, a switch network, a signal conditioning circuit, etc.) in accordance with the present disclosure. In aspects, the connector 210 may include a local microcircuit in accordance with the present disclosure. In aspects, the connector 210 may be coupled to an operator input device 214 (e.g., an injector, a foot pedal, an advancing slider, a torqueing mechanism, a recording button, an ablation button, etc.). In aspects, the connector 210 may be coupled to or include a control unit configured to accept one or more signals from the surgical tool 200, communicate one or more control signals thereto, send one or more pulsatile and/or radio frequency signals to the microcontroller, record one or more electrophysiological signals from the microsurgical tool, or the like.

In aspects, the control unit 210 (e.g., coupled to or included in the connector 210), may be connected to a display 216 configured to present one or more aspects of the recorded signals obtained at least in part with the surgical tool 200 to an operator, to present a map, at least partially dependent on the recorded signals, one or more metrics relating to the monitoring, one or more diagnostic test results, one or more stimulator test results, one or more electrophysiological maps, one or more neural structures to be preserved, etc.

In aspects, the connector 210 may be connected to an injector 214 (e.g., a manual high pressure injector, a syringe pump, a micro-injector, a power injector, etc.). The injector 214 coupled to a reservoir 212, the reservoir 212 configured to house a composition in accordance with the present disclosure prior to delivery to the target site 206.

In aspects, the system may include an imaging system 218, the imaging system may include an ultrasound element, a transducer, a piezoelectric element, an optical coherence tomography (OCT) element, a capacitive micromachined ultrasound transducer, a camera, an infrared camera, a near infrared camera, a deep tissue penetrating imaging element, an MRI, a CT system, or the like to image the tissues in the vicinity of the distal tip of the delivery device 200 during a procedure. Such elements may be advantageous for mapping, defining "keepout" zones, or monitoring tissues before, during or after a surgical procedure, monitoring migration of a composition after injection into the treatment site 206. Feedback from the elements may be advantageous for determining which nerves to spare and which nerves to treat as part of a procedure.

In aspects, the imaging system 218 may also be suitable for delivering ultrasound energy to one or more of the target tissues/features, as part of a treatment process (e.g., such as via a HIFU transducer, etc.). In one non-limiting example, the imaging system 218 may be configured to enable dual function imaging and sonication of a target site 206 in the body, (e.g., a vessel, innervated tissues, an organ, a ganglion, etc.), or between combinations thereof (i.e., an imaging/sonicating probe located in a first orifice and a guiding element, coupled element, etc. located in a second orifice).

In aspects, the imaging system 218 may be coupled 220 to the display 216 to provide visualization of the target site 206, monitor migration of a composition near the target site 206, overlay a physiologic signal over the image of the target site 206, etc.

In aspects, a procedure in accordance with the present disclosure may include inducing a partial or complete block of a neural signal, and/or receptor, augmentation of the function of a receptor, transmission of a neural signal (i.e., to/from a target organ), a partial and/or substantial neurectomy, peripheral neurectomy, sympathectomy, parasympathectomy, and the like.

In aspects, one or more systems in accordance with the present disclosure may be coupled with one or more imaging modalities including computer assisted imaging computed tomography (CT), magnetic resonance imaging (MRI), positron emission tomography (PET), optical coherence tomography (OCT), magnetoencephalography (MEG), functional MRI, stereotactic surgery, and the like before, during, and/or after a surgical procedure. Such imaging modalities may be included in the imaging system 218, and may be used to provide visualization 222 of a target tissue, of inflammation (e.g., inflammation as caused by an associated disease state, as caused by a procedure, etc.), of advancement of one or more aspects of the system towards the target tissue, etc. Use of such imaging modalities may be performed prior to/after surgery and/or intraoperatively.

In aspects, one or more distal tips or delivery elements of the delivery tool 200 in accordance with the present disclosure may include a fiber optic coupled to a laser (i.e., fiber optic guided radiation to a target tissue), a cryotherapy unit, a heat circulation unit (i.e., a unit for heated wire thermal therapy), an ultrasonic generator, or the like for treatment of target tissue. For purposes of discussion, the majority of non-limiting examples discussed herein are directed to electrical interfacing with tissues, ultrasonic interfacing with tissues, and chemical delivery aspects of such therapies.

A delivery system in accordance with the present disclosure may be configured such that at least a portion thereof may be placed into a lumen (e.g., an artery, a vein, an arteriole, a venule, a duct, a chamber, a pocket, a tubule, a bowel, a urethra, or the like), and/or an organ (e.g., a prostate, a testicle, a kidney, a stomach, a brain, a pancreas, a liver, a lung, or the like) so as to access the neural structure for purposes of diagnosis, and/or treatment of a disease state.

In aspects, the delivery tool 200 may include an elongate member and one or more probes (e.g., needles). One or more of the probes may be coupled to the elongate member. In aspects, at least one probe may be configured so as to slide-ably advance from the elongate member into the wall of a lumen adjacent thereto. The probe may be configured to interface with one or more target tissues in the wall, and/or with a volume of tissue externally positioned with respect to the wall. In aspects, the elongate member may be sized and dimensioned to be delivered via a lumen to the vicinity of a target tissue, the probes may then be advanced therefrom, through the wall of the lumen and into the target tissue in order to monitor, treat, diagnose a condition, or the like.

In aspects, the system may include a plurality of probes, the probes (e.g., needles) oriented so as to protrude from the elongate member during an actuation (i.e., a deployment or retraction of the probes from the elongate member, such actuation may be automatic, semi-automatic, manual, etc.). Each probe may be configured so as to be advance-able into a lumen wall adjacent thereto during a deployment procedure. One or more probes may be configured to communicate (e.g., fluidically communicate, electrically communicate, optically communicate, etc.) with the target tissues, with another device coupled to the body (e.g., an electrode, a surgical tool in accordance with the present disclosure, etc.), and/or between two or more probes.

In aspects, one or more probes may be arranged so as to be advanced, retracted, twisted, and/or actively bent (e.g., in the case of an active material based probe, a micro-wire actuated probe, etc.) either manually by an operator, or via a robotic actuation (e.g., a mechanism, a servo-controlled mechanism, etc.) during a deployment procedure. Such a configuration may be advantageous for assisting with placement of a probe during a procedure, with aligning a probe with a region of target tissue, advancing the probe through a target tissue, precisely placing one or more regions of the probe within a target tissue, etc.

In aspects, one or more probes may include a microneedle electrode, configured such that at least a portion thereof (e.g., a tip, a shank, a region, a plurality of regions, etc.) may be configured so as to facilitate electrical communication with one or more target tissues adjacent thereto, one or more probes, and/or one or more external electrodes as part of a deployment, monitoring, or treating procedure.

In aspects, a probe may include an array of electrodes, configured so as to assist with determination of a local field gradient, configured so as to monitor a plurality of sites along the length of the probe, to provide a configurable electrode arrangement for sensing, stimulation, ablation, etc.

In aspects, one or more electrodes may be arranged with an active area (i.e., area available to electrically interface with adjacent tissues) of less than 10mm², less than 1mm², less than 0.1mm², less than 10,000µm², less than 1,000µm², less than 100µm², less than 1µm², etc. Alternatively, one or more electrodes may be configured so as to form electrical impedance in normal saline of greater than 100ohm, greater than 1kohm, greater than 100kohm, greater than 1Mohm, greater than 10Mohm, greater than 50Mohm, etc.

In aspects, one or more probes may be configured with a characteristic width (i.e., a dimension perpendicular to a length measurement thereof, for example, a diameter), of less than 1mm, less than 200µm, less than 100µm, less than 50µm, less than 12µm, less than 3µm, etc. Such characteristic width may vary along the length of the probe. In aspects, one or more probes may be tapered to a fine tip (e.g., a tip with less than 5µm radius of curvature, less than 1µm radius of curvature, etc.) so as to more easily be advanced through tissues during a procedure.

In aspects, one or more regions of a probe or elongate member in accordance with the present disclosure may be coated with a substance and/or treated so as to be lubricious in the presence of water. Some non-limiting examples of such coatings include a hydrophilic coating, a silicone coating, a PTFE coating, parylene, a ceramic, PEBAX, a hydrogel, etc. Some non-limiting examples of such treatments include vapor deposition of a ceramic, a polymer, an ion treatment process, an electroplating process, dip process, etc. Such coating may provide for easier deployment as part of a surgical procedure in accordance with the present disclosure.

In aspects, one or more probes may include a tip fashioned with a tip electrode (e.g., an exposed region of the probe suitable for electrically interfacing with a surrounding tissue, with one or more probes, an external electrode, etc.). In aspects, the tip electrode may be arranged so as to provide a microscopic interface over a length at an end of the probe less than 150µm, less than 50µm, less than 20µm, less than 10µm, less than 1µm, and the like. Such a configuration may be suitable for spatially precise monitoring of local field potentials during a procedure (e.g., during monitoring of electrophysiological activity, during a denervation procedure, during placement of the probe, etc.). In aspects, the tip electrode may be arranged so as to provide an intermediately sized interface along the length of the probe, greater than 50µm but less than 1mm, greater than 100µm but less than 500µm, or the like. Such an arrangement may be suitable for stimulating local tissues, for monitoring overall electrophysiological activity around a volume of tissue, to act as a reference electrode, and the like. In aspects, the tip electrode may be configured along a length of the probe greater than 100µm, greater than 500µm, greater than 1mm, greater than 2mm, and the like. Such an arrangement may be advantageous for providing a sufficiently high current to surrounding tissues in the vicinity of the electrode, for example, during a hyperpolarizing stimulation, during an ablation procedure, to substantially affect tissues in the vicinity of the tip electrode, and the like.

In aspects an electrode in accordance with the present disclosure may be formed from an electrically and/or ionically conductive material. Some non-limiting examples of electrode materials include gold, platinum, platinum iridium, stainless steel, tungsten, iridium, palladium, rhodium, organic conducting polymer modified materials, poly(acetylene)s, poly(pyrrole)s, poly(thiophene)s, poly(terthiophene)s, poly(aniline)s, poly(fluorine)s, poly(3-alkythiophene)s, polytetrathiafulvalenes, polynapthalenes, poly(p-phenylene sulfide), poy(para-phenylenevinylene)s, poly(3,4-ethylenedioxythiophene) (PEDOT), poly(3,4-ethylenedioxythiophe) /poly(styrenesulfonate) (PEDOT/PSS), polyfuran, polyindole, polycarbazole, nanorods, nanotubules, carbon nanotubes, carbon fibers, combinations thereof, hybridized composites thereof, and the like. In one non-limiting example, an electrode in accordance with the present disclosure may include a PEDOT film hybridized with gold nanoparticles (e.g., gold particles with diameter less than 20nm, less than 15nm, etc.). In aspects, one or more electrodes may include a nanomaterial filler or functionalized material for enhancing one or more properties thereof (e.g., active area, conductivity, etc.).

In aspects, an electrode including an organic conducting polymer or a functionalized organic conducting polymer (e.g., via grafting of specie to the backbone thereof, grafting of an organometallic, biomolecule, etc. thereto, and the like) may be configured so as to monitor a local event associated with tissues in the vicinity of the electrode during use. In such a configuration, the electrical conductivity of the organic conducting polymer in contact with the surrounding tissues may change by orders of magnitude in response to pH, local potential changes, concentration of an analyte (e.g., a neurotransmitter, a neuroblocker, a neural agonist, a neural antagonist, an inverse agonist, an enzyme, a protein, oxygen, etc.) during use. Such changes may be advantageously monitored during a surgical procedure, so as to assess placement of the probe, determine progress of an associated treatment, or the like.

In aspects, one or more probes/needles may include a fluid delivery channel for delivery of a fluid (e.g., a medication, a stimulant, a neural agonist, a neural antagonist, an inverse agonist, a neuroblocker, a sclerosing alcohol, a neurotransmitter, a chemical denervation agent, a neurodisruptive agent, a sclerosing agent, phenol, alcohol, guanethidine, an antibody drug conjugate, etc.) for delivery to the target tissues. In one non-limiting example, one or more probes may include a microchannel for delivery of fluid. In an aspect associated with a method for treating a target tissue in accordance with the present disclosure, the system may be configured to deliver a bolus of a denervation agent to the target tissues. In aspects, the fluid may be delivered as part of a surgical procedure (e.g., nerve stimulation, denervation, chemical neurolysis, chemical neurolytic blockade, cryoablation, etc.).

A delivery system, comprising a delivery tool including a lumen, the lumen forming a fluid coupling between a distal end and a proximal end of the delivery tool, wherein the delivery tool including a lumen comprises a syringe with a needle; a reservoir for retaining a composition according to claims 1-7 prior to delivery of the composition to a treatment site within a volume of tissue, the reservoir coupled with the proximal end of the delivery tool; an injector coupled to the reservoir, the injector configured to deliver a bolus of the composition into the delivery tool upon activation thereof; and a delivery tip coupled to the lumen, the delivery tip deploy-ably coupled to the delivery tool, shaped and dimensioned so as to penetrate into or bias against the volume of tissue upon deployment from the delivery tool, the delivery tip comprising one or more ports coupled to the lumen, the ports arranged upon the delivery tip so as to access the treatment site.

Fig. 2b illustrates aspects of a delivery tool 224 in accordance with the present disclosure. The delivery tool 224 includes a catheter 226 including a thermal control element 228. The thermal control element 228 is configured to control the temperature of a composition passing through the catheter 226 towards a target site 230 in the body. The delivery tool 224 may include or couple to a connector 232, an injector 234, and/or a reservoir 236 each in accordance with the present disclosure. The delivery system may include an additional thermal control element 238 coupled to the reservoir 236 and/or the injector 234 to maintain a composition therein at a temperature during delivery 240 of the composition to the target site 230, during a procedure, prior to delivery 240, etc.

Fig. 2c illustrates a cross section A from Fig. 2b of a catheter 226. The catheter 226 is shown with a lumen 242 running substantially the length thereof, for delivery of a composition there through. A wall of the lumen 242 may be lubricated, and may include a lubricating substance (e.g., a PTFE liner, a silicone oil fluid, a hydrophilic layer, etc.) so as to help with passage of the composition there along during delivery to a target site in a body. The catheter 226 may include a heater band 244 to provide a thermal control function along the lumen, the heater band 244 configured to heat the lumen 242 so as to maintain a fluid therein at an elevated temperature. In aspects, the heater band 244 may include a resistive heating element (e.g., a resistive heating coil, etc.), a radio frequency (RF) heating element, a fluid transfer jacket, etc.

The catheter 226 may be constructed by traditional means (e.g., from an extruded tube, layered tubes, braided tube, coiled wire and tube, etc.). In aspects, the catheter 226 may be constructed in a layer by layer process. The process may include starting with a mandrel, the mandrel shaped so as to form the lumen, optionally a low friction or lubricious sheath placed over the mandrel, a first polymer layer coated onto the mandrel or sheath (e.g., via a solution casting method), the heating element added to the resulting composite (e.g., such as a laser cut hypotube, a resistive coil, reinforcing resistive braid, etc.), one or more additional polymer layers coated onto the heating element and first polymer layer, or one or more additional polymer layers (e.g., one or more insulating layers, etc.), may be coated onto the structure so as to form a thermally insulating layer between the heating element and an outer surface of the catheter.

The catheter 226 and the heater band 244 therein may be coupled to a thermal regulating unit 246/248, configured so as to control the temperature along the wall of the lumen 242 during use. In aspects, the lumen 242 may be maintained at a temperature of 40 - 50°C, of 43 - 47°C, etc. In aspects, a phase change composition in accordance with the present disclosure may be delivered through the catheter 226, the lumen 242 heated such that the phase change composition maintains a first state (e.g., a substantially low viscosity state), and upon delivery to the target site within a body, the phase change composition transitions to a second state (e.g., a gel state, a substantially high viscosity state, a solid state, etc.).

Fig. 2d shows a schematic of aspects of a delivery tool 250 in accordance with the present disclosure. The delivery tool 250 may include a lumen 255 arranged therein so as to couple a connector/controller 260 to a distal tip for delivery 280 of a composition in accordance with the present disclosure to a target site in a body. The delivery tool 250 may include one or more sensing regions 275, 285 for monitoring one or more electrophysiological signals, one or more physiologic parameters, or the like. In aspects, the delivery tool 250 may include one or more ablative zones 290, the ablative zone 290 optionally including a biasing function 295 (e.g., a balloon, a deployable region, a helical region, a shaped region, etc.) configured so as to bias against the walls of a vessel in the body during a procedure so as to deliver energy, a compound, inject a needle into, the wall of the vessel, etc. Also shown is an injector 265 coupled to the connector/controller 260, as well as a reservoir 270 coupled to the injector 265.

In aspects, the delivery tool 200, 224, 250 may be configured to deliver one or more diagnostic or stressing agents into a vessel in the body. Some non-limiting examples of such agents include neuro-stimulants, neuro-blockers, neuro-depressors, diuretics, hormones, steroids, nutrients, enzymes, biomarkers, antibodies, proteins, carbohydrates, analgesic, saline, plasma, combinations thereof or the like. The delivery of a stressing agent may be used in conjunction with the sensing to determine the organ response, a bodily response, etc. to the resulting stress state. Such delivery may be directed into an organ, a portion of an organ, a vessel wall serving an organ, into a ganglion, etc. in order to assess function and/or generate a stress response therefrom.

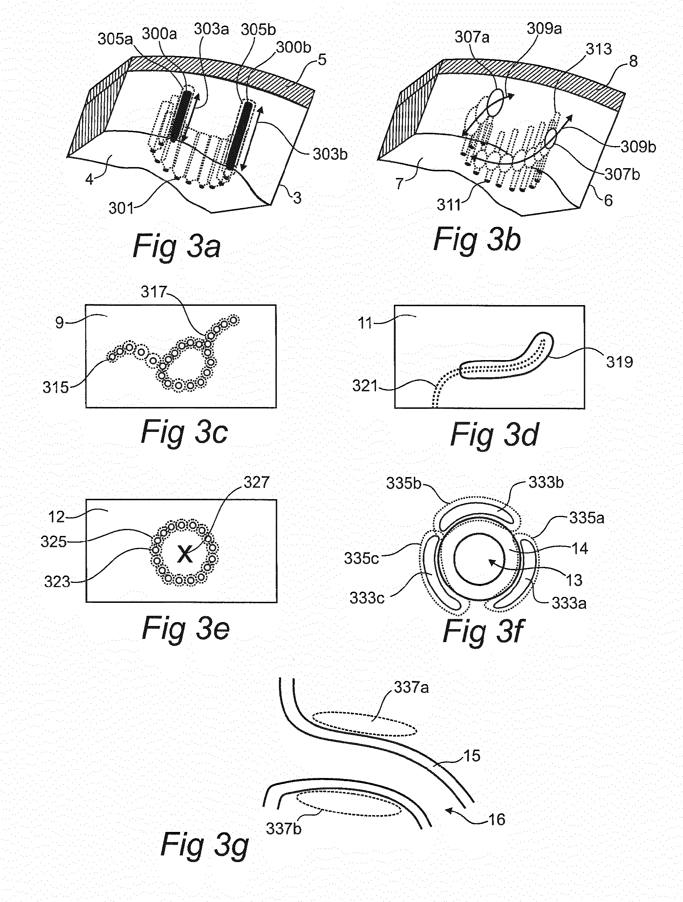

Figs. 3a3j show aspects of patterned delivery of a composition in accordance with the present disclosure to a volume of tissue.

Fig. 3a illustrates a volume of tissue 3 with an accessible face 4 through which a composition in accordance with the present disclosure has been injected, so as to form a pattern within the volume of tissue 3. The volume of tissue 3 may be associated with an organ tissue, adipose tissue, a vessel, a lumen wall, a muscle, a cardiac muscle, a brain tissue, an artery wall, a bowel wall, a bladder wall, etc. The composition is shown having been injected into the volume of tissue 3 through the accessible face 4 via one or more injection sites 301, each bolus 300a,b of the composition optionally shaped in accordance with the present disclosure (i.e., in this non-limiting example, shown as a post or elongated shape). In order to form a macroscopic shape in the volume of tissue 3, the boluses 300a,b may be formed via one or more injection sites 301, along a path within the volume of tissue 3, at coordinates within the volume of tissue 3, etc. As shown in Fig. 3a, the boluses 300a,b are formed as posts, each with a characteristic length 303a,b and width so as to form a substantially continuous fence around a region within the volume of tissue 3. In aspects, upon delivery to the volume of tissue 3 one or more components of the boluses 300a,b may migrate into the surrounding tissues, so as to form a zone of effect 305a,b. In aspects, the zone of effect 305a,b may be arranged (i.e., based on the migration of the desired component in the composition into the surrounding tissues, based on uptake into the tissues, etc.) such that an essentially continuous "structure" of effected tissues are formed in the volume of tissue 3. In aspects, the zone of effect 305a,b may be arranged such that isolated regions of tissue are affected by the treatment (i.e., such as around a vessel, within a tumor, around a diseased tissue, etc.).

In aspects, the volume of tissue 3 may include a region 5, which is not meant to be treated (e.g., a region of tissue that is meant to be preserved, a region that is not meant to substantially receive an active agent, etc.). Such a region 5 may be part of an adjacent organ, region of tissue on the existing organ that is functioning, a region that is susceptible to failure, provides a barrier function, etc.

Fig. 3b illustrates a volume of tissue 6 with an accessible face 7 into which an array of boluses 307a,b have been injected, so as to form one or more paths 309a,b through the volume of tissue 6, for treatment of the tissues in the immediate vicinity of the path 309a,b. In Fig. 3b, the pathways 309a,b are formed through multiple injections and delivery of boluses 307a,b at sites within the volume of tissue 6. The injections were made through injection sites 311 along the accessible face 7 of the volume of tissue 6. The needle tracks 313 for the injections are shown for clarity.

In aspects, more complex patterns, multiple paths 309a,b, etc. may be formed through a plurality of injections, such as placement of substantially spherical boluses, at sites in the 3D volume of tissue 6. Such an approach may be a-likened to a raster printed 3D shape, so as to form a barrier around a tumor margin, to follow a 3D pathway through a volume of tissue, etc.

Alternatively, additionally, or in combination, one or more of the paths 309a,b may be formed by passage of a needle through the volume of tissue 6, along a desired trajectory. The boluses 307a,b may be delivered either during insertion, pull back (such as with a delivery system having an end port on the needle for delivery), once the needles are placed (such as from a needle with multiple delivery ports, etc.), etc.

Fig. 3c illustrates a treatment pattern formed within a volume of tissue as seen from an accessible surface 9. The pattern is formed through a plurality of injections of boluses 315, which may migrate locally to form regions of treatment 317 around the boluses 315. The pattern may include linear regions (so as to form a fence like barrier in cardiac tissues, so as to follow along a vessel, so as to follow along a neural plexus, etc.), circular regions (so as to isolate a region of tissue from a region around it, to modify a conduction pathway through a volume of tissue, etc.).

Fig. 3d illustrates a treatment pattern formed within a volume of tissue as seen from an accessible surface 11. The pattern is formed by deposition of a bolus 319 of a composition in accordance with the present disclosure into the tissues along a pathway (e.g., a straight pathway, a curved pathway, a circular pathway, a tortuous pathway, etc.). As shown in Fig. 3d, the delivery needle injection pathway 321 is shown to further highlight the concept of shaping the bolus 319 to conform to a specific region within the tissues.

Fig. 3e illustrates a treatment pattern formed within a volume of tissue as shown from an accessible surface 12. The treatment pattern is formed during a series of injections 323 of a composition in accordance with the present disclosure, to form an effective treatment region 325, in this case the pattern formed in a circular shape so as to isolate a region 327 of the tissues from the surrounding tissues. Such an approach may be advantageous for altering the conduction of a bioelectrical signal through a muscle in the body, to isolate an asynchronous pacing center from nearby tissues, etc.

Fig. 3f illustrates a treatment pattern formed around a lumen 13 in a body, near to, through, and/or within a wall 14 of the lumen 13. The pattern is shown in a circumferential arrangement around the lumen 13. The boluses 333a-c of one or more compositions in accordance with the present disclosure have been injected into the tissues surrounding the lumen 13, in this case, so as to form a substantially complete ring around the lumen 13. The boluses 333a-c may have been injected through the wall 14 of the lumen 13 (i.e., from within the lumen), from an endoscopic approach (i.e., from outside the lumen 13), etc. One or more components of the composition in the boluses 333a-c may migrate so as to form a treatment zone 335a-c around the lumen 13. Such an approach may be advantageous for substantially forming a ring like treatment zone 335a-c around a lumen 13 in a body.

Fig. 3g illustrates an axial treatment pattern 337a,b formed along a vessel 15 in a body. The axial treatment pattern 337a,b may be formed through delivery of a composition through the wall of the vessel 15, such as via a delivery system in accordance with the present disclosure placed within a lumen 16 of the vessel 15. Such an axial treatment pattern 337a,b may be formed through multiple deliveries of boluses, through a shaped injection needle approach, or the like. Such an approach may be advantageous to limit regrowth of nerves along the walls of the vessel 15 after treatment thereof (i.e., so as to increase the durability of such a treatment).

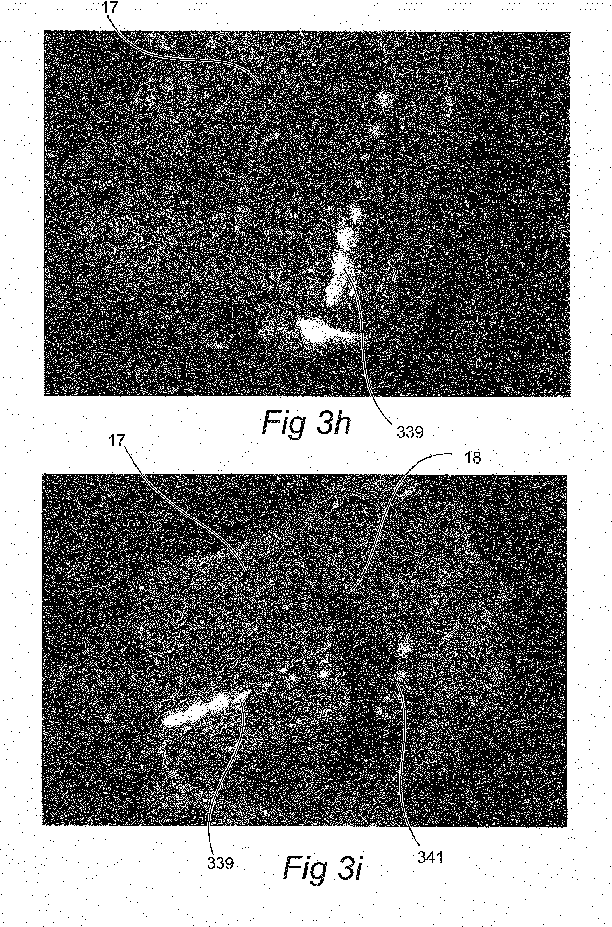

Fig. 3h shows a sample of muscle tissue 17 treated with a patterned example of a composition in accordance with the present disclosure. The composition is the same as described in Example 2, and was injected into the muscle tissue so as to form a wall of boluses in accordance with the present disclosure to form a series of boluses 339. The injections were made through a 25g stainless steel injection needle. The boluses 339 were formed by simultaneously injecting while retracting the injection needle from the tissues (i.e., so as to form an elongate bolus along the injection pathway). Alternatively, additionally, or in combination other approaches to forming the desired pattern in the tissue 17 may be employed in accordance with the present disclosure.

Fig. 3i shows the sample of muscle tissue 17 after being treated with a pattern of boluses 339 of a composition in accordance with the present disclosure. The muscle tissue 17 has been sliced 18 along a trajectory perpendicular to the pattern, so as to assess the width thereof post treatment. The width 341 of the "wall" pattern can be seen, wherein minimal lateral migration of the boluses 339 occurred post injection.

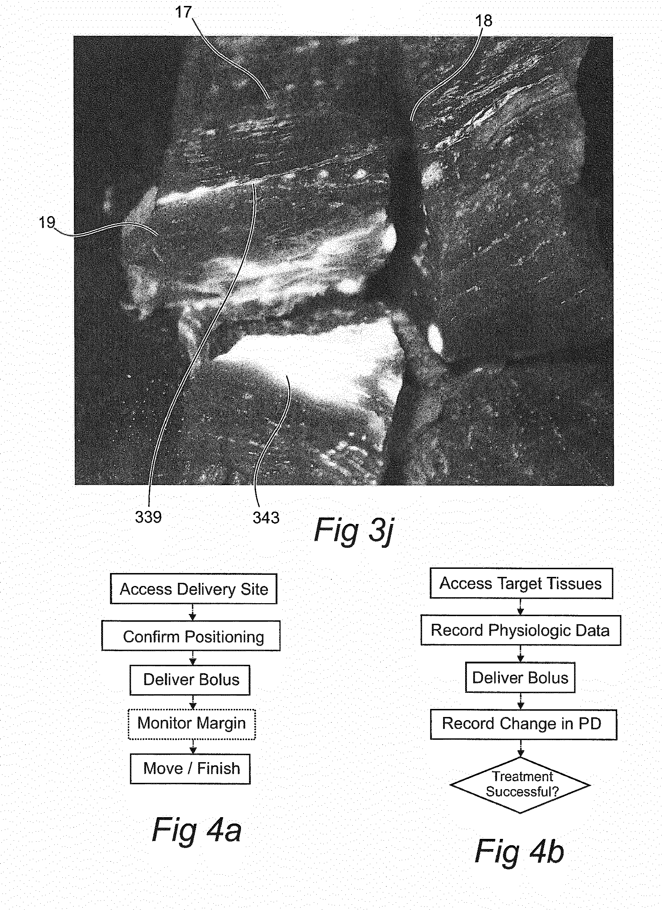

Fig. 3j shows the sample of muscle tissue 17 after being sliced 18 along a trajectory perpendicular to the pattern of boluses 339, and then sliced 19 again along the pattern of boluses 339. The second slice 19 illustrates how a substantially uniform treatment zone 343 was formed within the muscle tissue 17 around the pattern. Collectively Figs. 3h3j illustrate how a composition and injection method in accordance with the present disclosure may be used to form a patterned treatment zone within a volume of tissue 17 in a body.

Figs. 4a-4b show aspects of methods in accordance with the present disclosure. Fig. 4a shows aspects of methods for using a delivery system 200 in accordance with the present disclosure. Although the methods described include aspects for confirming treatment, monitoring margin, etc. they may be applied to treatment scenarios without substantial feedback steps. The method includes accessing a delivery site within a body, such as the parenchyma of an organ, a site along or through a vessel wall, or the like. By accessing the delivery site is meant coupling a tip or region of a delivery tool in accordance with the present disclosure with one or more anatomical sites within the body, so as to provide fluid communication between a reservoir and the anatomical sites for which treatment is desired. Such access may include delivery of a tool tip to a desired treatment site, deployment of one or more delivery needles towards the desired treatment site, to penetrate the wall of a lumen to access the treatment site, etc.

The method may optionally include confirming placement near the anatomical site, such as by recording physiologic activity from tissues in the vicinity thereof (e.g., with a sensor or electrode, a guidewire, a delivery tool, etc. each in accordance with the present disclosure), and monitoring a trend in the physiologic signal (e.g., during a stimulation event, during a stress test, etc.), making a diagnosis or prognosis based upon the recorded signal (e.g., a diagnosis of a disease state associated with local physiologic activity in the tissues, making a prognosis relating to an outcome of a disease state associated with activity in the tissues or tissues associated therewith, etc.), via direct imaging of the tissues with an imaging system in accordance with the present disclosure, etc. The method may include delivering a bolus of a composition in accordance with the present disclosure to the tissues, in the form of a pattern, etc. The method may include optionally monitoring the margin of a tissue target near the delivery site, and/or monitoring the migration of the composition or a component thereof upon delivery to the tissues. The method may include moving the delivery tool, retracting a delivery needle, or otherwise finishing the treatment by decoupling the delivery tool from the treatment site.

In aspects, the method may include one or more additional steps in accordance with the present disclosure. In aspects, the method may include placing an additional tool including one or more sensors and/or electrodes at a remote location (with respect to the organ) in the body and stimulating the local anatomy at either the remote site or within the parenchyma of the organ and monitoring an evoked response within the target tissues or at the remote site respectively. Such a configuration may be advantageous for elucidating information about the connectivity between the two sites (i.e., relevant to determining if a neuromodulation procedure applied there between has been successful, etc.).

Fig. 4b illustrates an additional method, the additional method including accessing the target tissues (alternatively an anatomical site of interest, a vessel, an artery, a vein, an arteriole, a venule, etc.), and recording and/or mapping the electrophysiological activity in the vicinity of the anatomical site of interest. The mapping may be provided by sweeping a sensory tip in accordance with the present disclosure over the anatomical site of interest, inserting and then withdrawing the sensory tip, deploying the sensory tip and then dragging and/or rotating the deployed tip along/around the lumen wall, combinations thereof, and the like. In aspects, the method may include displaying the mapped physiologic information for a user, constructing an anatomical model therefrom, directing a surgical robot to perform a treatment therefrom, comparing the map with a previously determined map (e.g., as a means for monitoring the outcome of a procedure, tracking a therapy, etc.), combinations thereof, or the like. In aspects, the method may include providing one or more directions to a surgeon and/or a surgical robot to access one or more regions of the mapped anatomy, overlaying the present map with previously generated maps (so as to evaluate changes in functionality, activity, etc.), combinations thereof, and the like.

The method may include delivering a bolus of a composition in accordance with the present disclosure to the target tissues, and optionally assessing an anatomical site of interest within the vicinity of the target tissues or coupled thereto, stimulating one or more physiologic systems in the body, and/or monitoring the evoked response at the anatomical site of interest to determine the effect of the bolus on the target tissues. The method may include recording a change in physiological data (PD). The method may include assessing the functionality of the anatomical site of interest, the site of stimulation (i.e., if the stimulation is of a localized type), the target tissues, or an anatomical site there between. The method may include assessing if the treatment was successful, such as via recording a marked change in neural traffic from affected tissues, a change in the proportion of neural response to a stress test, etc.

In aspects, the method may include ablating one or more anatomical sites within the body.

In aspects, one or more methods in accordance with the present disclosure may be completed, at least in part, with a delivery tool 200 in accordance with the present disclosure.

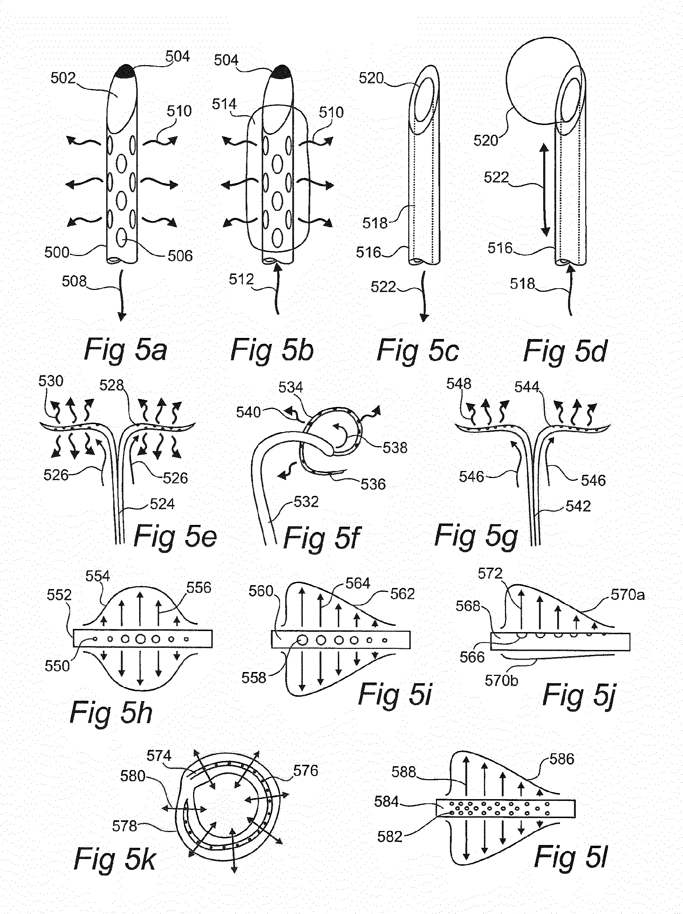

Figs. 5a-5l show aspects of delivery tips in accordance with the present disclosure.

Fig. 5a shows a needle like delivery tip 500 in accordance with the present disclosure to deliver a bolus of a composition in accordance with the present disclosure to a target tissue site within a body. The delivery tip 500 includes a plurality of ports 506 connected to a lumen within the delivery tip 500. The ports 506 may be arranged at one or more sites along the length of the delivery tip 500 so as to provide a particular shape to the bolus delivery, etc. The ports 506 may be distributed over the delivery tip 500, sized, and/or shaped so as to influence the bolus shape over the delivery tip 500. The delivery tip 500 is configured to accept the composition through the lumen from a coupled injector 508 during delivery. During delivery the composition is delivered 510 to the tissues through the ports 506. In aspects, the delivery tip 500 may include one or more sensors 504, electrodes, or the like to monitor local physiologic activity, monitor the movement or migration of the composition after injection, etc. In this non-limiting example, the sensor 504 is configured as an electrode, may include one or more exposed regions, each exposed region configured to interact with tissues and measure an electrophysiological signal therefrom. One or more of the sensors 504 may be configured in accordance with the present disclosure so as to assist in the guidance of the tip, measure local electrophysiological activity, determine bolus margins, determine when the tip is within a target tissue site, etc.

Fig. 5a shows a delivery tip 500 with a closed end 502, such that delivery of the bolus is made along the shank of the delivery tip 500.

Fig. 5b shows the tip from Fig. 5a after delivery 510, 512 of a bolus 514 of a composition in accordance with the present disclosure to a target tissue site surrounding the delivery tip 500. In this non-limiting example, the ports 506 are distributed and shaped such that the bolus 514 is substantially elongate in shape (e.g., sausage like, fence post like, cylindrical in shape, etc.).

Fig. 5c illustrates aspects of a delivery tip 516 in accordance with the present disclosure with a sharp tip, the sharp tip including a port 520, the delivery tip 516 including a lumen 518 in fluid communication with a proximal end thereof (e.g., a connector, a controller, an injector, etc.). The delivery tip 516 is configured to accept the composition through the lumen from a coupled injector 522 during delivery.

Fig. 5d illustrates the delivery tip 516 after delivery 518 of a bolus 520 of a composition in accordance with the present disclosure through the delivery tip 516 to a tissue site in a body. In this non-limiting example, the bolus 520 forms an essentially spherical shape upon delivery 518 to the tissues. In aspects, the position of the delivery tip 516 may be moved 522 so as to adjust the shape of the bolus 520 being delivered to the tissues. In aspects, the composition may include a contrast agent, so as to provide imaging of the injection site within the tissues. Movement 522 of the delivery tip 516 may be coordinated with the delivery 518 and the imaging in order to control the shape of the bolus 520 at the delivery site in the body.

Fig. 5e illustrates a curved delivery tip 524 in accordance with the present disclosure, configured so as to be advanced 526 into a volume of tissue in the body, the curvature providing a change in direction of the tip 524 so as to follow a path that is different than the initial direction of advancement within the tissues. The curved delivery tip 524 may include a plurality of ports 528 through which one or more boluses of a composition may be delivered 530 to the tissues. Such a configuration may be advantageous for forming a linear track of the composition within the tissues in a direction substantially different from the orientation of the delivery tip 524 to the tissues. Such a configuration may be advantageous for treating a linear track of tissues near to the surface of a volume of tissue, along a surface of a volume of tissue, etc.

Fig. 5f illustrates a delivery tip 532 in accordance with the present disclosure the delivery tip 532 including a deployable delivery member 534 (e.g., helically shaped, spiral shaped, circular shaped, elliptically shaped, etc.) configured such that the deployable delivery member 534 may take on a shape when deployed 538 from the delivery tip 532. In aspects, the deployable delivery member 534 may be shaped such that it can form a shape within a volume of tissue, or upon deployment within a lumen in a body (such that it can be biased against a wall of the lumen after deployment). In aspects, the deployable delivery member 534 includes a plurality of ports 536 arranged along the length thereof through which a composition in accordance with the present disclosure may be delivered 540 there through to a volume of tissue along a surface within a body, etc. Such a configuration may be advantageous to form a shaped delivery element that may be stably biased against a surface. The deployable delivery member 534 and the ports 536 arranged thereupon may be arranged such that the delivery 540 of the composition is substantially directed against a surface or along a side of the shape formed after deployment 538. Such a configuration may be advantageous to deliver a composition to a surface of a volume of tissue in a body.

Fig. 5g illustrates a curved delivery tip 542 in accordance with the present disclosure, configured so as to be advanced 546 into a volume of tissue in the body, the curvature providing a change in direction of the tip 542 so as to follow a path that is different than the initial direction of advancement within the tissues. The curved delivery tip 542 may include a plurality of ports 544 through which one or more boluses of a composition may be delivered 548 to the tissues. As shown in Fig. 5g the ports 544 are distributed on the tip 542 such that the composition would be delivered to a side thereof, such that if the tip 542 was biased towards a surface, a composition could be delivered thereto and dwell between the biased tip 542 and the surface so as to treat a region of the surface. Such a configuration may be advantageous for treating a linear track of tissues near to the surface of a volume of tissue, along a surface of a volume of tissue, etc.

Fig. 5h illustrates a profile of ports 550 arranged along a delivery tip 552 with varying characteristic width, such that delivery 556 of a composition therefrom forms an elliptical profile 554 (e.g., an egg like profile, a top like profile, elliptical lobes, etc.). In aspects, the profile 554 may take on a lobe like structure (such as pedals on a flower when looking axially down the axis of the delivery tip 552), etc. The ports 550 are shaped and arranged such that the larger diameter ports 550 are situated towards the center of the delivery region (the region around which the composition is delivered), while smaller diameter ports 550 are located near to the edges of the delivery region, so as to form the desired elliptical profile 554.

Fig. 5i illustrates a profile of ports 558 arranged along a delivery tip 560 with varying characteristic width, such that delivery 564 of a composition therefrom forms an conical profile 562 (e.g., an arrowhead-like profile, a pedal like conical profile, etc.). In aspects, the profile 562 may take on a lobe like structure (such as pedals on a flower when looking axially down the axis of the delivery tip 560), etc. The ports 558 are shaped and arranged such that the larger diameter ports 558 are situated towards one end of the delivery region (the region around which the composition is delivered), while smaller diameter ports 558 are located near to the other end of the delivery region, so as to form the desired conical profile 562.

Fig. 5j illustrates a profile of ports 566 arranged along a delivery tip 568 with varying characteristic width, such that delivery 572 of a composition therefrom forms a directed profile 570a, 570b (e.g., a profile where the delivery 572 is asymmetrically directed around the delivery tip 568 so as to preferentially deliver the composition to a side of the delivery tip 568). In aspects, the profile 570a, 570b may take on a lobe like structure (here being a single pedal or lobe to a side of the delivery tip 568), etc. The ports 566 are shaped and arranged along a side of the delivery tip 568 such that the larger diameter ports 566 are situated towards one end of the delivery region (the region around which the composition is delivered), while smaller diameter ports 566 are located near to the other end of the delivery region, so as to form the desired asymmetrically directed conical profile 570a, 570b.

Fig. 5k illustrates a profile of ports 576 arranged along a spiral shaped delivery tip 574 with varying characteristic width, such that delivery 580 of a composition therefrom forms a toroidal profile 578 (e.g., a donut like profile, a ring-like profile, shaped so as to isolate a region from the surrounding tissues, etc.). In aspects, the profile 578 may take on a beaded string like structure (such that individual boluses are arranged along the shape of the profile so as to form an undulating toroidal shape), etc. The ports 576 may be distributed, shaped, and/or arranged so as to alter the shape of the toroidal profile 578.

Fig. 5l illustrates a profile of ports 582 arranged along a delivery tip 584 with varying characteristic width, such that delivery 588 of a composition therefrom forms an conical profile 586 (e.g., an arrowhead-like profile, a pedal like conical profile, etc.). In aspects, the profile 586 may take on a lobe like structure (such as pedals on a flower when looking axially down the axis of the delivery tip 584), etc. The ports 582 may be distributed over the delivery tip 584 such that the density of the ports 582 is varied along the length thereof. In aspects, the ports 582 may be arranged such that a high density of ports are situated towards one end of the delivery region (the region around which the composition is delivered), while a lower density of ports 582 are located near to the other end of the delivery region, so as to form the desired conical profile 586.

In aspects, a delivery system or tool in accordance with the present disclosure may include a plurality of delivery tips each tip configured and arranged so as to contribute to a pattern of a composition in accordance with the present disclosure into a volume of tissue in a body. As such, macro patterns may be formed from a plurality of bolus deliveries, from a plurality of delivery tip deliveries, from delivery tips shaped so as to pass along a pathway through a volume of tissue, combinations thereof, etc.

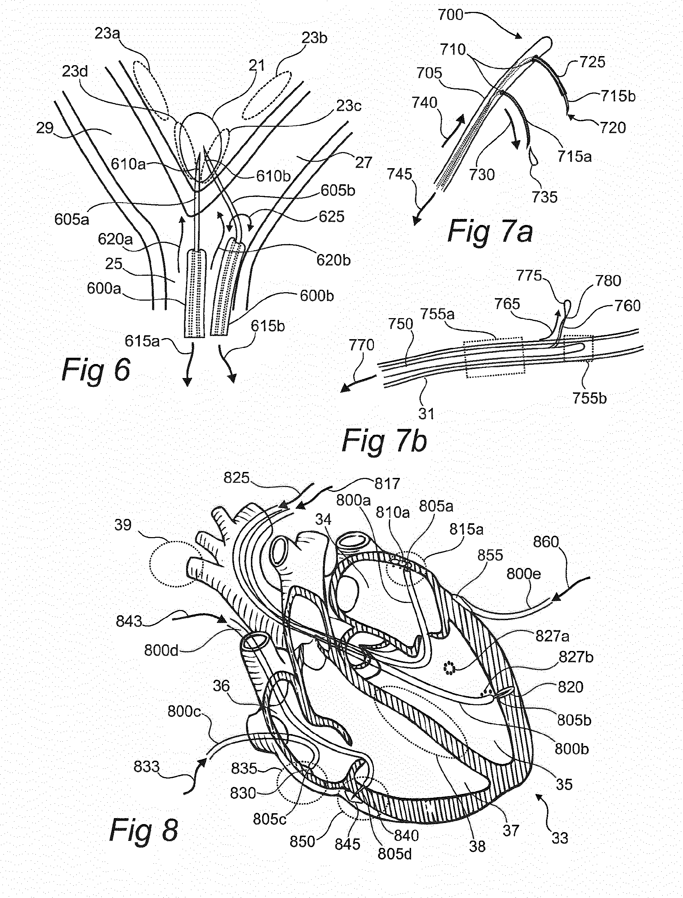

Fig. 6 shows application of a composition, delivery system, and delivery tool 600a,b each in accordance with the present disclosure to treatment of a carotid body 21 (i.e., a target site near to an access lumen such as a ganglion, a tumor, a sensory body, a node, a lymph node, etc.). The delivery tool 600a,b includes one or more needle-like delivery tips 605a,b in accordance with the present disclosure, each delivery tip 605a,b may be tipped with a sensor and/or electrode 610a,b each in accordance with the present disclosure. The delivery tip 605a,b may include a lumen to fluidly couple the distal tip of the delivery tool 600a,b to the proximal end thereof. The lumen may be coupled with one or more ports in accordance with the present disclosure so as to deliver a composition to the carotid body 21 or a site coupled thereto. The delivery tip 605a,b may be advanced 620a,b into the tissues around the carotid bifurication so as to couple one or more of the sensors and/or electrodes 610a,b with the carotid body 21 or one or more sites thereabout thus forming one or more target tissues, monitoring sites or treatment sites 23a - d within or around the carotid body 21. The device 600a, b may include a jacket to alter the stiffness of one or more segments of the device 600a,b, to protect the delivery tip 605a,b, one of the sensors 610a,b, etc. In aspects, the device 600a,b may include one or more stabilizing members, an anchor, a hook, a balloon, or the like, configured so as to stabilize and/or orient one or more regions of device 600a,b near to the intended treatment site. Once stabilized, the delivery tips 605a,b may be advanced 620a,b towards the carotid body 21 or an associated treatment site 23a - d. In aspects, the device 600a,b or associated delivery tip 605a,b may include one or more radiopaque markers, or may be constructed with one or more radiopaque materials in order to assist a surgeon with visualization of the surgical site during the procedure. In aspects, the stabilizing members may be configured to limit relative motion between the delivery tips 605a,b (e.g., the needles, the electrodes 610a,b, etc.) and the carotid body 21, vessel walls 25, 27, 29, associated treatment/monitoring sites 23a - d, etc. during one or more procedures performed thereon.

In aspects, the device 600a,b may be used to monitor one or more sites 23a - d within and around the carotid body 21 to assist in selectively ablating only a region of the carotid body (e.g., an outer layer, a surface, a chemoreceptor, a baroreceptor, etc.). In aspects, the device 600a,b may be used to both sense and selectively ablate and/or deliver a composition to regions of the carotid body 21 or a site 23a - d there about. In such procedures, the sensing may be performed with or without stimulation/stress to determine the ideal locations within the carotid body 21 to perform a neuromodulation, chemical denervation, ablation, delivery of a neural agonist, neural antagonist, etc. Upon determining the ideal locations, an RF current, a microbolus of neurotoxin, etc. may be injected into key sites amongst the monitoring/treatment sites 23a - d. Such a procedure may be advantageous for neuromodulating the carotid body 21 while limiting damage to surrounding structures, or to regions of the carotid body 21 that are to be spared in the procedure.

As shown in Fig. 6, the neural body 21 (such as, in this non-limiting example, a carotid body) may be located in the vicinity of a main carotid artery 25, an internal carotid artery 27, or an external carotid artery 29. The delivery tool 600a,b may be configured for placement in a lumen 25, 27, 29 in the vicinity of the neural body 21 (i.e., in this case a carotid body), neurons coupled thereto (in the vicinity of regions 23a - d), and/or receptors (i.e., in this case baroreceptors lining wall of the internal carotid artery 27). In aspects, one or more elements of the tool 600a,b may be configured so as to be actuate-ably advanced 620a,b into the wall of the lumen 25, 27, 29, or into contact therewith so as to be advanced towards a target tissue 23a - d (e.g., one or more regions of the neural body 21, a region adjacent to the neural body 23c,d, nerves and/or nerve plexuses 23a,b coupled to the neural body 21, and/or regions including receptors in the vicinity of the neural body 21 and/or the walls of the adjacent lumens 25, 27, 29, etc. The delivery tools 600a,b may be coupled with one or more controllers 615a,b respectively to manage needle deployment/retraction 620a,b, coupling of the delivery tips 605a,b or one or more sensors 610a,b with external electronics, a polygraph, or the like.

In aspects, one or more of the electrodes 610a,b may be configured to stimulate, and/or treat one or more regions of the carotid body 21, and/or one or more target tissues 23a - d as part of a surgical procedure. Additionally, alternatively, or in combination the delivery system may be configured to deliver a stressing agent (e.g., a hormone, a neurotransmitter, nitric oxide, oxygen, carbon dioxide, etc.) directly into the carotid body 21 to assess a change in the neural traffic assessed in the body 21 or within the vicinity of one or more of the target tissues 23a - d, assess a change in a body response to the stimulus (e.g., a change in heart rate, respiration, heart rate variability, blood pressure, sPO2, sympathetic outflow, mSNA changes, etc.). The region of treatment as well as the extent of treatment may be monitored and/or controlled by a circuit coupled with one or more electrodes on one or more of the delivery tips 605a,b.

In aspects, one or more electrodes 610a,b and/or delivery tips 605a,b may be configured to monitor, to stimulate, and/or to alter (e.g., deaden or block neural traffic, ablate the nerves, etc.), neurological activity in one or more nerve bundles extending from the neural body 21. Changes in neural traffic after a surgical procedure, in response to a stimulus, or the like may be used to assist in controllably treating one or more regions of target tissue 23c - d in or near the neural body 21, or other target tissues 23a - b in the vicinity thereof.

In aspects, an RF current may be applied through one or more of the electrodes 610a,b in order to treat the carotid body 21 or a target site 23a - d. The current may be passed between one or more of the electrodes 610a,b and a remotely located electrode (not explicitly shown) or between two or more of the electrodes 610a,b. Such a method may be advantageous for selectively controlling the current flow to the regions of the carotid body 21 in need of treatment. In aspects, the remotely located electrode may be a gel electrode placed upon the skin of the body (not explicitly shown), a needle electrode, an electrode placed within a nearby vein, or the like.

In aspects, a composition in accordance with the present disclosure may be injected into the carotid body 21. The composition may be formulated such that the ablation zone around the carotid body 21 is less than 5mm outside the margin of the carotid body, less than 3mm, less than 2mm, less than 1mm. Such adjustments may be made by altering the percentage of one or more excipients in the composition, adding a diluting agent (e.g., saline, water, etc.) to the composition, etc. In general, the composition may include a contrast agent in accordance with the present disclosure so as to visualize the migration of the composition after injection into the carotid body 21, or one or more treatment sites 23a - d coupled thereto.

In aspects, a method for treating such tissues may include injecting a first bolus of a first composition into or near to the carotid body 21, the first composition having an ablation and/or migration characteristic to treat at least a portion of the carotid body 21. The method including injecting one or more additional boluses of a second composition, the second composition having an ablation and/or migration characteristic suitable for treating another region of the carotid body 21, migrating outwards from the carotid body 21, etc.

In aspects, a method for treating a carotid body 21 may include accessing the arteriole vasculature of the carotid body and injecting a composition in accordance with the present disclosure into the vasculature, so as to fill the carotid body 21 with the composition. After injection, the composition will temporarily occlude blood flow within the carotid body 21 while the ablative component thereof diffuses into the tissues of the organ and completes ablation thereof (e.g., so as to ablate all receptors in the organ, to ablate particular receptor types in the organ, to ablate chemical receptors, to ablate baroreceptors, etc.). Such a method may be advantageous to safely treat the carotid body with minimal collateral damage to surrounding tissues. As the composition may quickly breakdown in the general blood flow, the risks to the subject are minimized, with ablation being very controllably delivered only to the tissues in the carotid body 21 that are intimately served by the vasculature thereof.

Figs. 7a-7b show aspects of a delivery system in accordance with the present disclosure for treating tissues along a vessel. Fig. 7a shows aspects of a delivery tool 700 for use in a delivery system in accordance with the present disclosure. The delivery tool 700 includes a jacket 705 including a plurality of ports 710 through which a plurality of delivery tips 715a,b in accordance with the present disclosure may pass through in order to couple with a local anatomical site of interest, to stabilize the delivery tip, etc. The delivery tips 715a,b may include one or more electrodes 720 and/or sensors at the tip thereof in order to interface with the local anatomical site of interest (e.g., to measure local electrophysiological activity, to determine placement of the tip, to determine if the tip has exited the lumen, etc.). In aspects, the delivery tips 715a,b may include an insulating layer 725 configured so as to isolate one or more aspects of the delivery tip 715b from the surroundings. In aspects, the insulating layer 725 may include a varying thickness, optionally arranged so as to form one or more step transitions along the length of the delivery tip 715b. Such steps may be advantageous for limiting the depth of penetration of the delivery tip 715b into the local tissues.

In aspects, the delivery tips 715a,b may include a lumen through which to deliver 730 a composition 735, a chemical substance, a medicament, etc. to the site of interest. The delivery tips 715a,b may include one or more ports, shaped elements, etc. in accordance with the present disclosure to treat a region of tissues, interact with an adjacent volume of tissue in a particular pattern, etc. In aspects, the delivery tips 715a,b may be deployed 740 from the delivery tool 700 so as to interact with an adjacent volume of tissue.

In aspects, the delivery tips 715a,b and/or anchors may be slidingly coupled with the jacket 705 such that they may be advanced 740 as part of a deployment procedure. In aspects, the delivery tips 715a,b and/or stabilizing elements may be coupled with a connector, actuator, and/or a controller 745 generally situated at the proximal end of the delivery tool 700.

Fig. 7b illustrates aspects of a delivery tool 750 in accordance with the present disclosure placed within a lumen 31. The delivery tool 750 may include one or more zones 755a,b in accordance with the present disclosure. The delivery tool 750 includes a first sensing zone 755a located along the length thereof for interfacing with the lumen 31 wall proximally to a treatment site. The delivery tool 750 includes a second sensing zone 755b located at the distal tip thereof for interfacing with the lumen 31 distally to a treatment site. The delivery tool 750 includes one or more microneedle delivery tips 760, which may be advanced from the body of the delivery tool 750 and into the wall of the lumen 31 into which it has been placed as part of a procedure. Such needle advancement or retraction 765 may be coordinated by an operator, a controller 770, etc. In aspects, the microneedle delivery tips 760 may provide a means for delivering a composition, a chemical agent 775 into the tissues surrounding the lumen 31. In aspects, the microneedle delivery tips 760 may include one or more electrodes 780 to monitor and/or interface (e.g., stimulate, ablate, etc.) with the local tissues upon deployment therein, to monitor (e.g., via impedance changes, via changes in local electrophysiological signals, etc.) a margin of migration or treatment of a bolus delivered to the tissues. In aspects, the delivery tool 750 may be configured so as to deliver the microneedle tips 760 into the adventitia of the lumen 31, or optionally directly into the parenchyma of an organ to be treated. Such a configuration may be advantageous to provide a composition in accordance with the present disclosure, a neurotoxin, a cancer treating agent, a neuroblocking agent, a neurostimulating agent, etc. into the target tissues as part of a treatment procedure in accordance with the present disclosure.

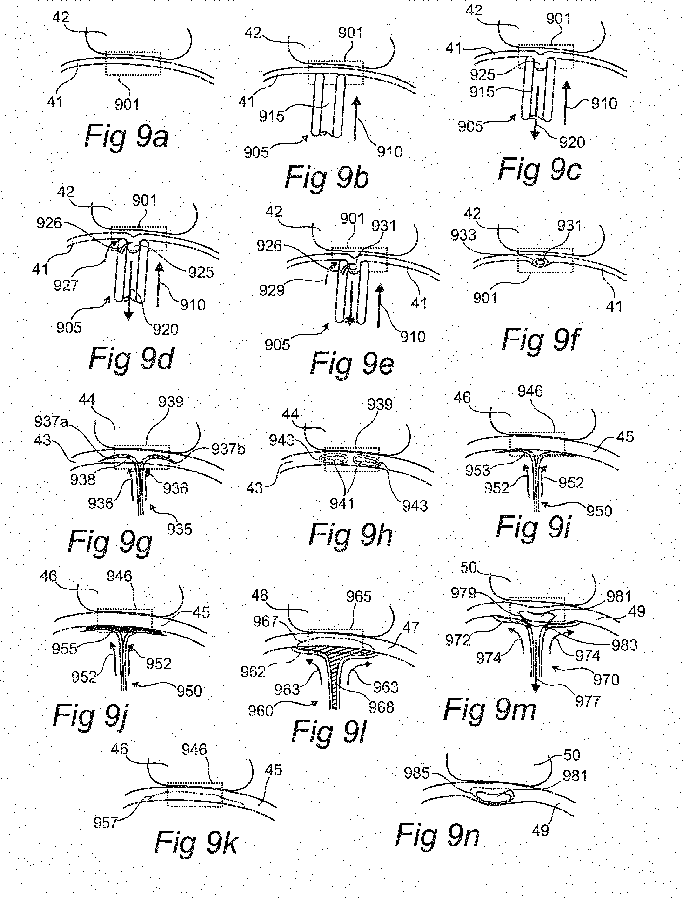

Fig. 8 shows aspects of systems and methods for treating cardiac tissue in accordance with the present disclosure. Fig. 8 illustrates a heart 33 of a subject, and the placement and interaction of delivery tools 800a - e with cardiac tissues of the heart in accordance with the present disclosure. A delivery tool 800a in accordance with the present disclosure is shown accessing the left atrium 34 of the heart 33 through the aorta, the delivery tool 800a coupled to the wall of the left atrium 34, a needle-like delivery tip 805a in accordance with the present disclosure interfacing with the wall, a plurality of boluses 810a of a composition in accordance with the present disclosure delivered 817 through the delivery tool 800a and deposited into the wall of the left atrium 34 around a desired treatment zone 815a. In aspects, the delivery tool 800a may include tissue capture means such as illustrated in Figs. 9a-9n so as to limit the treatment zone 815a to just the wall of the left atrium 34 (so as to limit collateral damage to nearby organs, to prevent perforation of the esophagus, etc.).

A delivery tool 800b is shown coupled with the wall of the left ventricle 35 of the heart 33, the delivery tool 800b including a delivery tip 805b penetrating into the wall of the left ventricle 35, a bolus 820 of a composition in accordance with the present disclosure delivered 825 through the delivery tool 800b and into the wall of the left ventricle 35 (such as forming a pattern in accordance with the present disclosure). A plurality of previously injected delivery sites 827a,b are shown in the left ventricle, demonstrating patterning of the boluses so as to treat zones of the tissue in accordance with the present disclosure. In aspects, the delivery tip 805b may be advanced into the pericardium of the heart 33 so as to treat neural structures, cardiac muscle, etc. in that region (i.e., passing from the interior of the heart through the wall and into the external tissue sites).

A delivery tool 800c in accordance with the present disclosure is shown interfacing with the right atrium 36 of the heart 33, the delivery tool 800c advanced through the inferior or superior vena cava (entering the body through the basilic vein, the femoral vein, etc.), a delivery tip 805c biased against the wall of the right atrium 36, a bolus 830 of a composition in accordance with the present disclosure having been delivered 833 to the wall, the composition dwelling against the wall so as to treat a site thereof within a treatment zone 835 along the wall.

A delivery tool 800d in accordance with the present disclosure is shown interfacing with the right ventricle 37 of the heart 33, the delivery tool 800d advanced through the inferior or superior vena cava (entering the body through the basilic vein, the femoral vein, etc.), the tip thereof biased against the wall and a delivery tip 805d advanced into the wall, such that a tip is placed near to the pericardium of the heart, so as to interact with an autonomic nerve, a pericardial site, etc. One or more sensing elements 840 (sensors, electrodes, etc.) may be incorporated into the delivery tool 800d, or delivery tip 805d, in accordance with the present disclosure, to guide the tip for delivering 843 a bolus 845, to monitor electrophysiological activity before, during, and/or after delivery of the bolus 845, to assess the margin of the bolus 845, etc. in the vicinity of a treatment zone 850.

A delivery tool 800e in accordance with the present disclosure may be delivered to the pericardial sac or space of the heart 33 (e.g., such as endoscopically, transcutaneously, during surgery, etc.). The delivery tool 800e may be aligned with a treatment site and a bolus 855 of a composition in accordance with the present disclosure may be delivered 860 thereto to treat one or more tissues sites on or near the pericardium of the heart.

In aspects, a delivery tool 800a - e in accordance with the present disclosure may be used to access one or more treatment sites along, into, or in the vicinity of the vein of Marshall, the septum 38, a carotid sinus 39, a carotid body, the posterior left atrium, the great cardiac vein, the coronary sinus, the left superior cardinal vein, the oblique vein, the venous valve of Vieussens, etc.

A delivery tool 800a - e may include a sensor, an electrode, etc. in accordance with the present disclosure to assess the effect of the treatment, to assist with guiding the delivery tool 800a - e to the neural targets (e.g., via measuring local neural traffic, via stimulation of local tissues, etc.), assist with the assessment of margins of the bolus (e.g., by assessing impedance changes around the sensors, assessing the neural, and/or epicardial traffic around the sensors, etc.).

In aspects, a delivery tool in accordance with the present disclosure may include a plurality of tips, one or more deployable tips or tip arrays, etc. so as to treat a wide swath of tissues, to rapidly form a treatment pattern, etc. in the tissues.

Figs. 9a-9n show aspects of a delivery system and method for treating tissues in a thin walled structure. Fig. 9a shows a thin walled section 41 (e.g., a wall of an atrium, a bowel wall, a bladder wall, an esophagus wall, a membrane, a vaginal wall, a pericardial sac, etc.) and an adjacent structure 42 that is not to be treated (e.g., an esophagus beside an atrial wall, a prostate next to a bladder, a gall bladder next to a duodenum, etc.). The desired treatment zone 901 is shown substantially within the thin walled section 41.

Fig. 9b illustrates aspects of a delivery tool 905 in accordance with the present disclosure, the delivery tool 905 biased 910 against the thin walled section 41 so as to seal a lumen 915 against the wall and the tip of the delivery tool 905.

Fig. 9c illustrates application of a vacuum, or suction 920 to the lumen 915 of the delivery tool 905 to draw a section of tissue 925 into the lumen 915. Such an approach may be advantageous to confidently capture and retain the tissue segment for subsequent treatment thereof. In aspects, the tip of the delivery tool 905 may include a plurality of electrodes (not explicitly shown), for passing an RF current through the section of tissue 925, so as to safely treat it without affecting the adjacent structure 42.

Fig. 9d illustrates the delivery tool 905, having drawn a section of tissue 925 into the lumen 915 thereof, the delivery tool 905 driving, engaging, or otherwise penetrating 927 a microneedle delivery tip 926 in accordance with the present disclosure into the section of tissue 925, so as to engage therewith.

Fig. 9e illustrates delivery 929 of a bolus 931 of a composition in accordance with the present disclosure into the section of tissue 925, the composition retained within the section of tissue 925 for treatment thereof.

In aspects, the tip of the delivery tool 905 may include one or more electrodes in accordance with the present disclosure to assess the electrophysiological properties of the tissues, to assess the effect of the bolus on the tissues, etc.

Fig. 9f illustrates the thin walled section 41 after removal of the delivery tool 905, the bolus 931 embedded therein, one or more active components of the bolus 931 diffusing into the tissues to form a treatment zone 933. The adjacent structure 42 is substantially untreated, unpenetrated, etc. Such an approach may be advantageous for precisely treating thin walls without penetrating them, without affecting adjacent structures 42, etc.

Fig. 9g shows a delivery tool 935 in accordance with the present disclosure including two delivery tips 937a,b having been advanced 936 into a thin walled section 43 without penetrating there through or into an adjacent structure 44. The delivery tips 937a,b include a plurality of ports 938 for delivery of a composition there through into the thin walled section 43.

Fig. 9h shows a plurality of boluses 941 after injection by the delivery tool 935 of Fig. 9g after the tool has been retracted from the thin walled section 43. One or more active elements of the composition have diffused into the adjacent tissues to form a local treatment zone 943 within the thin walled section 43 but without substantially affecting the adjacent structure 44. In aspects, the local treatment zone 943 is the region into which the initial boluses 941 will migrate after injection into the local tissues. The extent of the local treatment zone 943 is determined by the properties of the composition delivered, the local tissue properties, and the like.

Fig. 9i illustrates a delivery tool 950 biased 952 against a thin walled section 45, the delivery tool 950 including a plurality of ports 953 arranged thereupon such that the ports 953 are in intimate contact with the thin walled section 45 upon biasing 952 the device there against. The thin walled section 45 is near to an adjacent structure 46 for which treatment is not desired (treatment may generally be desired in the treatment zone 946).

Fig. 9j shows the delivery tool 950 after delivery of a bolus 955 of a composition in accordance with the present disclosure to the interface between the ports 953 and the thin walled section 45. The tool 950 may be held against the tissues for a period of time, such that the composition may treat the tissues, such that one or more components of the composition may diffuse into the tissues, etc.

Fig. 9k shows the thin walled section 45 and a treated zone 957 substantially in the desired treatment zone 946, having treated the thin walled section 45 without substantially affecting the adjacent structure 46.

Fig. 9l illustrates a delivery tool 960 with a deployable fixture 962, the deployable fixture shaped like an inverted umbrella, a suction cup, etc., the deployable fixture 962 shown after deployment 963 within a lumen of a body, the deployable fixture biased against a thin walled section 47. The thin walled section 47 includes a desired treatment zone 965 substantially residing within the thin walled section 47 and outside of the margins of an adjacent structure 48. The delivery tool 960 is shown with a bolus 968 of a composition in accordance with the present disclosure biased against the thin walled structure 47 so as to form a treatment zone 967 substantially aligned with the desired treatment zone 965.

Fig. 9m shows a delivery tool 970 with a deployable fixture 972 deployed and biased 974 against a thin walled section 49. The delivery tool 970 includes a lumen in which a vacuum 977 has been formed so as to draw a section of the thin walled structure 49 onto one or more delivery tips 979 in accordance with the present disclosure. After interfacing the delivery tips 979 with the thin walled structure 49, one or more boluses 981 of a composition in accordance with the present disclosure may be injected into the section for treatment thereof. In aspects, the delivery tips 979 or deployable fixture 972 may include one or more sensors, electrodes, etc. 983 to record electrophysiological activity, detect contact with the wall, monitor delivery of the boluses 981 into the thin walled section 49, monitor the resulting treatment process, monitor changes in electrophysiological activity in the adjacent tissues, etc.

Fig. 9n shows the thin walled section 49 and the adjacent structure 50 with the embedded boluses 981 of composition, the composition forming a treatment zone 985 substantially within the thin walled section 49.

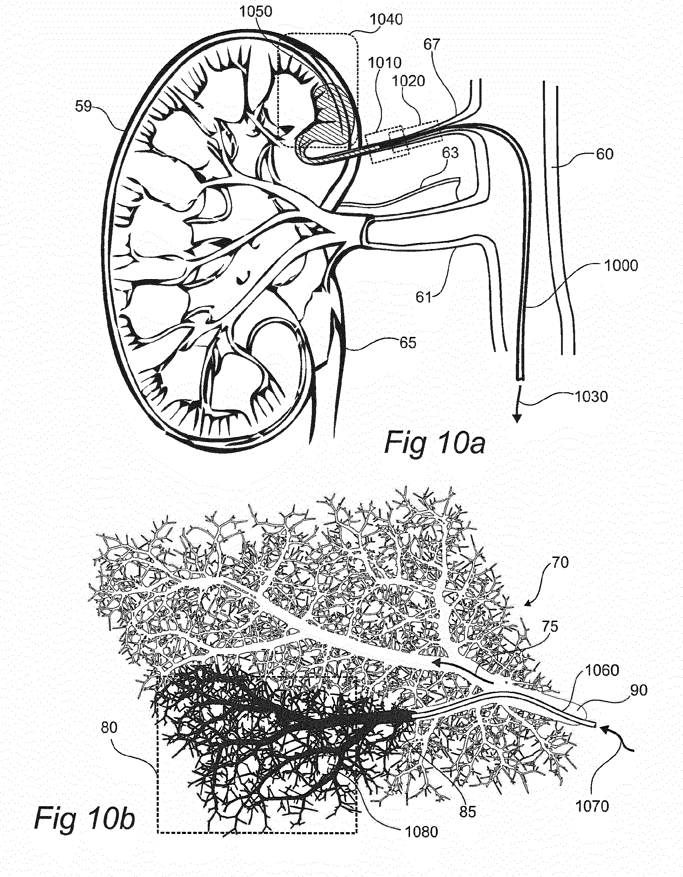

Figs. 10a-10b show schematics of aspects of a delivery system and composition for treating a volume of tissues in an organ in a body in accordance with the present disclosure. Fig. 10a shows a kidney 59 with a renal artery 61, a renal vein 63, a ureter 65 and an accessory artery 67, the renal artery 61 and the accessory artery 67 coupled to the aorta 60 of a subject. A schematic depicting a distal tip of a delivery tool 1000 in accordance with the present disclosure is shown positioned within the accessory artery 67, having been routed through the aorta 60. The delivery tool 1000 optionally including one or more monitoring zones 1010, 1020 including one or more sensors and/or ablation components in accordance with the present disclosure, the delivery tool 1000 including a distal tip with a delivery tip at zone 1010 in accordance with the present disclosure configured to deliver a bolus 1050 of a composition in accordance with the present disclosure into the accessory artery 67. The delivery tool 1000 may be coupled 1030 to a connector, a controller, an injector, a reservoir, etc. The bolus 1050 may be delivered into the accessory artery 67 to treat one or more sites there along zones 1010, 1020 and/or to treat a region 1040 of the kidney 59 coupled to the accessory artery 67. In aspects, the bolus may be configured so as to dwell in the arteries and arterioles coupled to the accessory artery 67 so as to restrict oxygen to the tissues served by those arteries, to deliver a component of the compound to the tissues served by the accessory artery 67, to ablate the tissues adjacent to the accessory artery 67, to deliver a toxin, neurotoxin, cytotoxin, etc. to a tissue site in an organ, a combination thereof, or the like.

In aspects, the approach described herein may be applied to the embolization of tissues in the vicinity of a tumor, to treat diseased tissues in an organ, or the like.

Fig. 10b shows a vascular tree 70 and/or a lymphatic tree (e.g., a tree of vessels within an organ, within a volume of tissue, an arterial tree within an organ, etc.) with fluid (e.g., blood, lymph, bile, etc.) flowing 75 through the vessels, a delivery tool 1060 in accordance with the present disclosure has been inserted up the main artery 90 of the tree and a bolus 1080 of a composition in accordance with the present disclosure has been delivered 1070 into a branch 85 (e.g., a target vessel) of the tree 70, the branch substantially exclusively providing fluid flow to a target region 80 (e.g., a region of the organ served by the branch of the arterial tree, etc.). The bolus 1080 may travel throughout the target vessel 85 and more distal branches therefrom so as to treat the vessel and/or tributaries thereof. Such an approach may be advantageous for treating vessels and one or more branches thereof, for ablating one or more nerves travelling on the vessel and/or branches thereof, for embolizing and ablating diseased tissues or tumor tissues in an organ, to perform a controlled release of an ablative agent into the walls of a vessel and/or branches thereof, combinations thereof, or the like.

In aspects, a method for treating and/or assessing tissues in the vicinity of the target region 80 may include delivering a composition and/or stressing agent each in accordance with the present disclosure into the branch 85 so as to treat and/or stress tissues in the target region 80. In aspects, one or more physiological parameters of the tissue, the target region 80, the vessel tree 70, the main vessel 90, the branch 85, the organ, or a systemic process relating to the stress test, may be monitored before, during, and/or after the stress test so as to determine the stress/functional relationship of the target region 80.

Some non-limiting examples of stressing agents include a vasodilator, a vasoconstrictor, a neuroblocker, a neurostimulant, a neural antagonist, a neural agonist, an inverse agonist, a diuretic, insulin, glucose, beta-adrenergic receptor antagonist, angiotensin-11 converting enzyme inhibitor, calcium channel blocker, an HMG-CoA reductase inhibitor, digoxin, an anticoagulant, a diuretic, a beta blocker, an ACE inhibitor, a steroid, a combination thereof, or the like.

## Claims

1. A composition, comprising:
an ablative agent comprising ethyl alcohol for performing a treatment within a body of a subject, wherein the ablative agent represents more than 85% of the composition by mass;
an excipient for limiting migration of at least one of the composition and the ablative agent within the body after delivery to the site, wherein the excipient comprises hydroxypropyl cellulose having an average molecular weight of approximately 1,000,000 or greater; and
one or more surfactants;
wherein the composition exhibits a change from a first set of properties in a first local environment comprising a syringe with a needle to a second set of properties in a second local environment comprising the tissue site; and
wherein the first set of properties comprises a first viscosity and the second set of properties comprises a second viscosity, the second viscosity being higher than the first viscosity; and
wherein the composition forms a low viscosity fluid at a temperature between 45 and 80° Celsius, and has a low shear rate viscosity of greater than 1000 mPa-s (cPs).

2. The composition in accordance with claim 1, wherein:
the composition forms a viscous thixotropic gel with a thixotropic index of greater than 1.25 at 37° Celsius.

3. The composition in accordance with claim 1, wherein:
the composition forms a Bingham plastic with a yield strength of greater than 5 Pascals at 37° Celsius.

4. The composition in accordance with claim 1, wherein:
the one or more surfactants are selected from the group consisting of sodium lauryl sulfate, ammonium lauryl sulfate, lauryl alcohol ether sulfate, and trimethylcoco ammonium chloride.

5. The composition in accordance with claim 1, wherein the first viscosity of the composition is less than 100 mPa.s (cps) at a first temperature range of 45 to 50°C, and the second viscosity of the composition is greater than 500 mPa.s (cps) at a second temperature range of 35 to 40°C.

6. The composition in accordance with claim 1, wherein the ablative agent forms a vehicle for the composition, the viscosity of the composition increasing as the active agent migrates into a volume of tissues surrounding the site, after delivery to the site.

7. The composition in accordance with claim 1, wherein the composition is formulated so as to limit migration of the active agent from an injection site to a distance of less than approximately 3mm from a margin of a bolus formed by the composition after delivery to the site within a timeframe comparable with the delivery of the composition to the site.

8. A delivery system, comprising:
a delivery tool including a lumen, the lumen forming a fluid coupling between a distal end and a proximal end of the delivery tool, wherein the delivery tool including a lumen comprises a syringe with a needle;
a reservoir for retaining a composition according to claims 1-7 prior to delivery of the composition to a treatment site within a volume of tissue, the reservoir coupled with the proximal end of the delivery tool;
an injector coupled to the reservoir, the injector configured to deliver a bolus of the composition into the delivery tool upon activation thereof; and
a delivery tip coupled to the lumen, the delivery tip deploy-ably coupled to the delivery tool, shaped and dimensioned so as to penetrate into or bias against the volume of tissue upon deployment from the delivery tool, the delivery tip comprising one or more ports coupled to the lumen, the ports arranged upon the delivery tip so as to access the treatment site.

9. The delivery system in accordance with claim 8, further comprising a thermal regulating unit coupled to at least one of the lumen and the reservoir, the thermal regulating unit configured to maintain the composition at a predetermined temperature at least one of prior to delivery and during delivery.

10. The delivery system in accordance with claim 8, wherein the ports are arranged along the delivery tip with at least one of a spatially changing density and a spatially changing diameter such that the bolus may be shaped when delivered from the delivery tip.

11. The delivery system in accordance with claim 8, further comprising a tissue suction element, coupled to the delivery tip, the suction element configured to retain a site against the delivery tip upon activation at least one of before the delivery, during the delivery, and after the delivery.

12. The delivery system in accordance with claim 11, wherein the suction element is configured to draw the site onto the delivery tip upon activation and wherein the delivery tip is arranged within the suction element so as to deliver the bolus into the drawn in site of the tissue.

13. A delivery system according to any of the claims 8-12 for use in a method for treating a region in a volume of tissue; and at least one of monitoring the effect of the composition on an electrophysiological state of a region in the volume of tissue and monitoring the migration of the composition in the region after delivery to the site.

14. A delivery system for use in accordance with claim 13, wherein delivering the composition to the tissue site comprises forming a pattern of the composition in the region.

15. A delivery system for use in accordance with claim 14, wherein the pattern is formed through deposition of a plurality of boluses at points over a three dimensional path within the volume of tissue.

## Patentansprüche

1. Komposition, umfassend:
ein Ablationsmittel umfassend Ethylalkohol zur Durchführung einer Behandlung innerhalb eines Körpers eines Subjekts, wobei das Ablationsmittel mehr als 85% der Masse der Zusammensetzung ausmacht;
einen Hilfsstoff zur Begrenzung der Migration von mindestens einem von der Komposition und dem Ablationsmittel innerhalb des Körpers nach Abgabe an die Stelle, wobei der Hilfsstoff Hydroxypropylcellulose mit einem durchschnittlichen Molekulargewicht von etwa 1.000.000 oder mehr umfasst; und
ein oder mehrere Tenside;
wobei die Komposition eine Änderung von einem ersten Satz von Eigenschaften in einer ersten lokalen Umgebung, die eine Spritze mit einer Nadel umfasst, zu einem zweiten Satz von Eigenschaften in einer zweiten lokalen Umgebung aufweist, die die Gewebestelle umfasst; und
wobei der erste Satz von Eigenschaften eine erste Viskosität und der zweite Satz von Eigenschaften eine zweite Viskosität umfasst, wobei die zweite Viskosität höher als die erste Viskosität ist; und
wobei die Komposition bei einer Temperatur zwischen 45 und 80° Celsius eine niedrigviskose Flüssigkeit bildet und eine niedrige Scherratenviskosität von mehr als 1000 mPa-s (cPs) aufweist.

2. Zusammensetzung nach Anspruch 1, wobei
die Zusammensetzung ein viskoses thixotropes Gel mit einem Thixotropieindex von mehr als 1,25 bei 37° Celsius bildet.

3. Zusammensetzung nach Anspruch 1, wobei
die Zusammensetzung einen Bingham-Kunststoff mit einer Streckgrenze von mehr als 5 Pascal bei 37° Celsius bildet.

4. Zusammensetzung nach Anspruch 1, wobei
die einen oder mehrere Tenside gewählt sind aus der Gruppe bestehend aus Natriumlaurylsulfat, Ammoniumlaurylsulfat, Laurylalkoholethersulfat und Trimethylcocoammoniumchlorid.

5. Zusammensetzung nach Anspruch 1, wobei die erste Viskosität der Zusammensetzung geringer ist als 100 mPa.s (cps) in einem ersten Temperaturbereich von 45 bis 50°C und die zweite Viskosität der Zusammensetzung größer ist als 500 mPa.s (cps) in einem zweiten Temperaturbereich von 35 bis 40°C.

6. Zusammensetzung nach Anspruch 1, wobei das Ablationsmittel ein Vehikel für die Zusammensetzung bildet, wobei die Viskosität der Zusammensetzung zunimmt, wenn das Wirkmittel nach der Abgabe an die Stelle in ein die Stelle umgebendes Volumen von Geweben migriert.

7. Zusammensetzung nach Anspruch 1, wobei die Zusammensetzung so formuliert ist, dass die Migration des Wirkmittels von einer Injektionsstelle zu einem Abstand von weniger als etwa 3 mm von einem Rand eines durch die Zusammensetzung gebildeten Bolus nach der Abgabe an die Stelle innerhalb eines mit der Abgabe der Zusammensetzung an die Stelle vergleichbaren Zeitrahmens begrenzt ist.

8. Verabreichungssystem, umfassend:
ein Verabreichungswerkzeug mit einem Lumen, wobei das Lumen eine Flüssigkeitskopplung zwischen einem distalen Ende und einem proximalen Ende des Verabreichungswerkzeugs bildet, wobei das Verabreichungswerkzeug mit einem Lumen eine Spritze mit einer Nadel umfasst;
ein Reservoir zum Zurückhalten einer Zusammensetzung nach den Ansprüchen 1 bis 7 vor der Abgabe der Zusammensetzung an eine Behandlungsstelle innerhalb eines Gewebevolumens, wobei das Reservoir mit dem proximalen Ende des Verabreichungswerkzeugs gekoppelt ist;
einen Injektor, der mit dem Reservoir gekoppelt ist, wobei der Injektor so konfiguriert ist, dass er bei Betätigung einen Bolus der Zusammensetzung in das Verabreichungswerkzeug abgibt; und
eine mit dem Lumen gekoppelte Verabreichungsspitze, wobei die Verabreichungsspitze ausfahrbar mit dem Verabreichungswerkzeug gekoppelt ist, so geformt und dimensioniert ist, dass sie beim Ausfahren aus dem Verabreichungswerkzeug in das Gewebevolumen eindringt oder gegen dieses vorgespannt ist, wobei die Verabreichungsspitze einen oder mehrere mit dem Lumen gekoppelte Anschlüsse aufweist, wobei die Anschlüsse an der Verabreichungsspitze so angeordnet sind, dass sie Zugang zur Behandlungsstelle haben.

9. Verabreichungssystem nach Anspruch 8, ferner umfassend eine Wärmeregulierungseinheit, die mit mindestens einem von dem Lumen und dem Reservoir gekoppelt ist, wobei die Wärmeregulierungseinheit so konfiguriert ist, dass sie die Zusammensetzung vor der Verabreichung und während der Verabreichung auf einer vorbestimmten Temperatur hält.

10. Verabreichungssystem nach Anspruch 8, wobei die Anschlüsse entlang der Verabreichungsspitze mit mindestens einem von einer räumlich veränderlichen Dichte und einer räumlich veränderlichen Durchmesser angeordnet sind, so dass der Bolus bei der Abgabe von der Verabreichungsspitze geformt werden kann.

11. Verabreichungssystem nach Anspruch 8, ferner umfassend ein Gewebeabsaugelement, das mit der Verabreichungsspitze gekoppelt ist, wobei das Absaugelement so konfiguriert ist, dass es bei Betätigung eine Stelle gegen die Einbringspitze hält bei mindestens einem von vor der Abgabe, während der Abgabe und nach der Abgabe.

12. Verabreichungssystem nach Anspruch 11, wobei das Saugelement so konfiguriert ist, dass es die Stelle bei Aktivierung auf die Verabreichungsspitze zieht, und wobei die Verabreichungsspitze innerhalb des Saugelements so angeordnet ist, dass der Bolus in die eingezogene Stelle des Gewebes abgegeben wird.

13. Verabreichungssystem nach einem der Ansprüche 8 bis 12 zur Verwendung in einem Verfahren zur Behandlung einer Region in einem Gewebevolumen; und
mindestens einem von einer Überwachung der Wirkung der Zusammensetzung auf einen elektrophysiologischen Zustand einer Region im Gewebevolumen und einer Überwachung der Migration der Zusammensetzung in der Region nach Abgabe an die Stelle.

14. Verabreichungssystem zur Verwendung nach Anspruch 13, wobei die Abgabe der Zusammensetzung an die Gewebestelle die Bildung eines Musters der Zusammensetzung in der Region umfasst.

15. Verabreichungssystem zur Verwendung nach Anspruch 14, wobei das Muster durch Abscheidung einer Vielzahl von Boli an Punkten über einem dreidimensionalen Pfad innerhalb des Gewebevolumens gebildet wird.

## Revendications

1. Composition, comprenant :
un agent ablatif comprenant de l'alcool éthylique et destiné à effectuer un traitement à l'intérieur d'un corps d'un sujet, dans laquelle l'agent ablatif représente plus de 85 % de la composition en masse ;
un excipient destiné à limiter la migration d'au moins l'un parmi la composition et l'agent ablatif à l'intérieur du corps après administration sur le site, dans laquelle l'excipient comprend de l'hydroxypropylcellulose ayant un poids moléculaire moyen d'environ ou supérieur à 1000000 ; et
un ou plusieurs tensioactifs,
dans laquelle la composition présente une modification en passant d'un premier ensemble de propriétés dans un premier environnement local comprenant une seringue dotée d'une aiguille, à un deuxième ensemble de propriétés dans un deuxième environnement local comprenant le site tissulaire,
dans laquelle le premier ensemble de propriétés présente une première viscosité et le deuxième ensemble de propriétés présente une deuxième viscosité, la deuxième viscosité étant supérieure à la première viscosité, et
dans laquelle la composition forme un fluide de faible viscosité à une température comprise entre 45 et 80 °C, et présente une viscosité à faible taux de cisaillement qui est supérieure à 1000 mPa.s (cP).

2. Composition selon la revendication 1, dans laquelle :
la composition forme un gel thixotrope visqueux avec un indice thixotropique supérieur à 1,25 à la température de 37 °C.

3. Composition selon la revendication 1, dans laquelle :
la composition forme un fluide de Bingham avec une limite d'élasticité supérieure à 5 Pa à la température de 37 °C.

4. Composition selon la revendication 1, dans laquelle :
lesdits un ou plusieurs tensioactifs sont choisis dans le groupe constitué du laurylsulfate de sodium, du laurylsulfate d'ammonium, de l'éthersulfate d'alcool laurique, et du chlorure de cocotriméthylammonium.

5. Composition selon la revendication 1, dans laquelle la première viscosité de la composition est inférieure à 100 mPa.s (cP) sur une première plage de températures de 45 à 50 °C, et la deuxième viscosité de la composition est supérieure à 500 mPa.s (cP) sur une deuxième plage de températures de 35 à 40 °C.

6. Composition selon la revendication 1, dans laquelle l'agent ablatif forme un véhicule pour la composition, la viscosité de la composition augmentant à mesure que l'agent actif migre dans un volume de tissu entourant le site, après administration sur le site.

7. Composition selon la revendication 1, dans laquelle la composition est formulée de manière à limiter la migration de l'agent actif depuis un site d'injection jusqu'à une distance inférieure à environ 3 mm d'une marge d'un bolus formé par la composition après administration sur le site dans un délai comparable à l'administration de la composition sur le site.

8. Système d'administration, comprenant :
un outil d'administration incluant une lumière, la lumière formant un couplage fluidique entre une extrémité distale et une extrémité proximale de l'outil d'administration, dans lequel l'outil d'administration incluant une lumière comprend une seringue dotée d'une aiguille ;
un réservoir destiné à retenir une composition selon les revendications 1 à 7 avant l'administration de la composition sur un site de traitement à l'intérieur d'un volume de tissu, le réservoir étant couplé à l'extrémité proximale de l'outil d'administration ;
un injecteur couplé au réservoir, l'injecteur étant configuré pour administrer un bolus de la composition dans l'outil d'administration lors de son activation ; et
un embout d'administration couplé à la lumière, l'embout d'administration étant couplé à l'outil d'administration de manière déployable, et étant formé et dimensionné de manière à pénétrer dans ou presser contre le volume de tissu lors de son déploiement depuis l'outil d'administration, l'embout d'administration comprenant un ou plusieurs orifices couplés à la lumière, les orifices étant disposés sur l'embout d'administration de manière à accéder au site de traitement.

9. Système d'administration selon la revendication 8, comprenant en outre une unité de régulation thermique couplée à au moins l'un parmi la lumière et le réservoir, l'unité de régulation thermique étant configurée pour maintenir la composition à une température prédéterminée avant et/ou pendant l'administration.

10. Système d'administration selon la revendication 8, dans lequel les orifices sont disposés le long de l'embout d'administration avec au moins l'un parmi une densité variable dans l'espace et un diamètre variable dans l'espace, de telle sorte que le bolus puisse être façonné lorsqu'il est administré depuis l'embout d'administration.

11. Système d'administration selon la revendication 8, comprenant en outre un élément d'aspiration de tissu qui est couplé à l'embout d'administration, l'élément d'aspiration étant configuré pour retenir un site contre l'embout d'administration lors de l'activation avant et/ou pendant et/ou après l'administration.

12. Système d'administration selon la revendication 11, dans lequel l'élément d'aspiration est configuré pour aspirer le site vers l'embout d'administration lors de l'activation, et dans lequel l'embout d'administration est disposé à l'intérieur de l'élément d'aspiration de manière à administrer le bolus dans le site aspiré du tissu.

13. Système d'administration selon l'une quelconque des revendications 8 à 12 à utiliser dans un procédé consistant à :
traiter une région dans un volume de tissu ; et
surveiller l'effet de la composition sur un état électrophysiologique d'une région dans le volume de tissu et/ou surveiller la migration de la composition dans la région après administration sur le site.

14. Système d'administration à utiliser selon la revendication 13, dans lequel l'administration de la composition sur le site tissulaire consiste à former un motif de la composition dans la région.

15. Système d'administration à utiliser selon la revendication 14, dans lequel le motif est formé par dépôt d'une pluralité de bolus en des points le long d'un trajet tridimensionnel à l'intérieur du volume de tissu.
